# EUROPEAN PATENT APPLICATION

(11) **EP 4 047 095 A1**
(43) Date of publication of application: **24.08.2022**
(21) Application number: 21158366.1
(22) Date of filing: 22.02.2021
(51) Int. Cl.: C12N 15/113

(54) **METHOD AND COMPOSITION FOR A TARGETED GENE KNOCKOUT**

(71) Applicant: Eberhard Karls Universität Tübingen Medizinische Fakultät, 72074 Tübingen (DE)
(72) Inventor: Skokowa, Julia, 72127 Kusteringen (DE); Nasri, Masoud, 72074 Tübingen (DE); Mir, Perihan, 70771 Leinfelden-Echterdingen (DE)
(74) Representative: Witte, Weller & Partner Patentanwälte mbB

(57) **Abstract**

The present invention relates to a composition for the targeted knockout of a gene on double-stranded DNA in a biological cell, a method for the targeted knockout of a gene on double-stranded DNA in a biological cell, a preparation comprising a biological cell prepared *in vitro,* said biological cell comprises a gene on double-stranded DNA, which is knocked-out in a targeted manner, a kit for the targeted knockout of a gene on double-stranded DNA in a biological cell, a method of treating a subject afflicted with a disease associated with a mutated gene, nucleic acid molecules which may be a component of said composition and methods, and a method of treating a subject afflicted with a disease associated with a mutated gene.

SEQUENCE LISTING

<110>  Eberhard-Karls-Universität Tübingen, Medizinische Fakultät

<120>  Method and composition for a targeted gene knockout

<130>  5402P621EP

<160>  258

<170>  PatentIn version 3.5

<210>  1
<211>  20
<212>  DNA
<213>  Artificial Sequence

<220>
<223>  None

<400>  1
gggctataag aggagccggg                                                    20


<210>  2
<211>  20
<212>  DNA
<213>  Artificial Sequence

<220>
<223>  None

<400>  2
gaggccgttg cattgcccca                                                    20


<210>  3
<211>  20
<212>  DNA
<213>  Artificial Sequence

<220>
<223>  None

<400>  3
ctcactttca cccaggctca                                                    20


<210>  4
<211>  20
<212>  DNA
<213>  Artificial Sequence

<220>
<223>  None

<400>  4
cggggaactc actttcaccc                                                    20


<210>  5
<211>  20
<212>  DNA

<213>  Artificial Sequence

<220>
<223>  None

<400>  5
ggtgaaagtg agttccccgt                                                   20


<210>  6
<211>  20
<212>  DNA
<213>  Artificial Sequence

<220>
<223>  None

<400>  6
gaaagtgagt tccccgttgg                                                   20


<210>  7
<211>  20
<212>  DNA
<213>  Artificial Sequence

<220>
<223>  None

<400>  7
ctcgtctgtt gcctccaacg                                                   20


<210>  8
<211>  20
<212>  DNA
<213>  Artificial Sequence

<220>
<223>  None

<400>  8
cctcgtctgt tgcctccaac                                                   20


<210>  9
<211>  20
<212>  DNA
<213>  Artificial Sequence

<220>
<223>  None

<400>  9
tcctcgtctg ttgcctccaa                                                   20


<210>  10
<211>  20
<212>  DNA
<213>  Artificial Sequence

<220>

<223>  None

<400>  10
cccgttggag gcaacagacg                                                   20


<210>  11
<211>  20
<212>  DNA
<213>  Artificial Sequence

<220>
<223>  None

<400>  11
tggaggcaac agacgaggag                                                   20


<210>  12
<211>  20
<212>  DNA
<213>  Artificial Sequence

<220>
<223>  None

<400>  12
ggcaacagac gaggagagga                                                   20


<210>  13
<211>  20
<212>  DNA
<213>  Artificial Sequence

<220>
<223>  None

<400>  13
acagacgagg agaggatgga                                                   20


<210>  14
<211>  20
<212>  DNA
<213>  Artificial Sequence

<220>
<223>  None

<400>  14
cgaggagagg atggaaggcc                                                   20


<210>  15
<211>  20
<212>  DNA
<213>  Artificial Sequence

<220>
<223>  None

<400>  15

agggctcatt cttgggggcc                                                    20

<210>  16
<211>  20
<212>  DNA
<213>  Artificial Sequence

<220>
<223>  None

<400>  16
acctcagggc tcattcttgg                                                    20

<210>  17
<211>  20
<212>  DNA
<213>  Artificial Sequence

<220>
<223>  None

<400>  17
aacctcaggg ctcattcttg                                                    20

<210>  18
<211>  20
<212>  DNA
<213>  Artificial Sequence

<220>
<223>  None

<400>  18
gaacctcagg gctcattctt                                                    20

<210>  19
<211>  20
<212>  DNA
<213>  Artificial Sequence

<220>
<223>  None

<400>  19
tgaacctcag ggctcattct                                                    20

<210>  20
<211>  20
<212>  DNA
<213>  Artificial Sequence

<220>
<223>  None

<400>  20
gcccccaaga atgagccctg                                                    20

<210>  21
<211>  20
<212>  DNA
<213>  Artificial Sequence

<220>
<223>  None

<400>  21
cagccgctcc ctgaacctca                                                    20


<210>  22
<211>  20
<212>  DNA
<213>  Artificial Sequence

<220>
<223>  None

<400>  22
ccagccgctc cctgaacctc                                                    20


<210>  23
<211>  20
<212>  DNA
<213>  Artificial Sequence

<220>
<223>  None

<400>  23
aagaatgagc cctgaggttc                                                    20


<210>  24
<211>  20
<212>  DNA
<213>  Artificial Sequence

<220>
<223>  None

<400>  24
agaatgagcc ctgaggttca                                                    20


<210>  25
<211>  20
<212>  DNA
<213>  Artificial Sequence

<220>
<223>  None

<400>  25
gagccctgag gttcagggag                                                    20


<210>  26
<211>  20
<212>  DNA

<213>  Artificial Sequence

<220>
<223>  None

<400>  26
cctgaggttc agggagcggc                                                   20


<210>  27
<211>  20
<212>  DNA
<213>  Artificial Sequence

<220>
<223>  None

<400>  27
agggagcggc tggagtgagc                                                   20


<210>  28
<211>  20
<212>  DNA
<213>  Artificial Sequence

<220>
<223>  None

<400>  28
gctggacgga gatctggggc                                                   20


<210>  29
<211>  20
<212>  DNA
<213>  Artificial Sequence

<220>
<223>  None

<400>  29
cgcagctgga cggagatctg                                                   20


<210>  30
<211>  20
<212>  DNA
<213>  Artificial Sequence

<220>
<223>  None

<400>  30
ccgcagctgg acggagatct                                                   20


<210>  31
<211>  20
<212>  DNA
<213>  Artificial Sequence

<220>

<223>  None

<400>  31
cccgcagctg gacggagatc                                                   20


<210>  32
<211>  20
<212>  DNA
<213>  Artificial Sequence

<220>
<223>  None

<400>  32
ctctgggacc cgcagctgga                                                   20


<210>  33
<211>  20
<212>  DNA
<213>  Artificial Sequence

<220>
<223>  None

<400>  33
cccagatctc cgtccagctg                                                   20


<210>  34
<211>  20
<212>  DNA
<213>  Artificial Sequence

<220>
<223>  None

<400>  34
ccagatctcc gtccagctgc                                                   20


<210>  35
<211>  20
<212>  DNA
<213>  Artificial Sequence

<220>
<223>  None

<400>  35
aggcctctgg gacccgcagc                                                   20


<210>  36
<211>  20
<212>  DNA
<213>  Artificial Sequence

<220>
<223>  None

<400>  36

cgtccagctg cgggtcccag                                                    20

<210>  37
<211>  20
<212>  DNA
<213>  Artificial Sequence

<220>
<223>  None

<400>  37
cgagtgtaac ccaggcctct                                                    20

<210>  38
<211>  20
<212>  DNA
<213>  Artificial Sequence

<220>
<223>  None

<400>  38
agctgcgggt cccagaggcc                                                    20

<210>  39
<211>  20
<212>  DNA
<213>  Artificial Sequence

<220>
<223>  None

<400>  39
gcgagtgtaa cccaggcctc                                                    20

<210>  40
<211>  20
<212>  DNA
<213>  Artificial Sequence

<220>
<223>  None

<400>  40
gctgcgggtc ccagaggcct                                                    20

<210>  41
<211>  20
<212>  DNA
<213>  Artificial Sequence

<220>
<223>  None

<400>  41
aggagctgcg agtgtaaccc                                                    20

<210>  42
<211>  20
<212>  DNA
<213>  Artificial Sequence

<220>
<223>  None

<400>  42
tgggttacac tcgcagctcc                                                    20


<210>  43
<211>  20
<212>  DNA
<213>  Artificial Sequence

<220>
<223>  None

<400>  43
gggttacact cgcagctcct                                                    20


<210>  44
<211>  20
<212>  DNA
<213>  Artificial Sequence

<220>
<223>  None

<400>  44
ggttacactc gcagctcctg                                                    20


<210>  45
<211>  20
<212>  DNA
<213>  Artificial Sequence

<220>
<223>  None

<400>  45
gttacactcg cagctcctgg                                                    20


<210>  46
<211>  20
<212>  DNA
<213>  Artificial Sequence

<220>
<223>  None

<400>  46
acactcgcag ctcctggggg                                                    20


<210>  47
<211>  20
<212>  DNA

<213>  Artificial Sequence

<220>
<223>  None

<400>  47
gcacgtcaag ggcctccccc                                                   20


<210>  48
<211>  20
<212>  DNA
<213>  Artificial Sequence

<220>
<223>  None

<400>  48
tgggaactga ggcacgtcaa                                                   20


<210>  49
<211>  20
<212>  DNA
<213>  Artificial Sequence

<220>
<223>  None

<400>  49
ttgggaactg aggcacgtca                                                   20


<210>  50
<211>  20
<212>  DNA
<213>  Artificial Sequence

<220>
<223>  None

<400>  50
gggttcctgt ttgggaactg                                                   20


<210>  51
<211>  20
<212>  DNA
<213>  Artificial Sequence

<220>
<223>  None

<400>  51
acgtgcctca gttcccaaac                                                   20


<210>  52
<211>  20
<212>  DNA
<213>  Artificial Sequence

<220>

<223>  None

<400>  52
ccttcccagg gttcctgttt                                                   20


<210>  53
<211>  20
<212>  DNA
<213>  Artificial Sequence

<220>
<223>  None

<400>  53
tccttcccag ggttcctgtt                                                   20


<210>  54
<211>  20
<212>  DNA
<213>  Artificial Sequence

<220>
<223>  None

<400>  54
cagttcccaa acaggaaccc                                                   20


<210>  55
<211>  20
<212>  DNA
<213>  Artificial Sequence

<220>
<223>  None

<400>  55
agttcccaaa caggaaccct                                                   20


<210>  56
<211>  20
<212>  DNA
<213>  Artificial Sequence

<220>
<223>  None

<400>  56
cccaaacagg aaccctggga                                                   20


<210>  57
<211>  20
<212>  DNA
<213>  Artificial Sequence

<220>
<223>  None

<400>  57

cacttctctg gtccttccca                                                    20

<210>  58
<211>  20
<212>  DNA
<213>  Artificial Sequence

<220>
<223>  None

<400>  58
gcacttctct ggtccttccc                                                    20

<210>  59
<211>  20
<212>  DNA
<213>  Artificial Sequence

<220>
<223>  None

<400>  59
ctgcgcaata ggcacttctc                                                    20

<210>  60
<211>  20
<212>  DNA
<213>  Artificial Sequence

<220>
<223>  None

<400>  60
tcgggcactc actgcgcaat                                                    20

<210>  61
<211>  20
<212>  DNA
<213>  Artificial Sequence

<220>
<223>  None

<400>  61
cggccacatg cagctgtgtc                                                    20

<210>  62
<211>  20
<212>  DNA
<213>  Artificial Sequence

<220>
<223>  None

<400>  62
ccggccacat gcagctgtgt                                                    20

<210>  63
<211>  20
<212>  DNA
<213>  Artificial Sequence

<220>
<223>  None

<400>  63
gtgcccgaca cagctgcatg                                                    20


<210>  64
<211>  20
<212>  DNA
<213>  Artificial Sequence

<220>
<223>  None

<400>  64
ccgacacagc tgcatgtggc                                                    20


<210>  65
<211>  20
<212>  DNA
<213>  Artificial Sequence

<220>
<223>  None

<400>  65
tacccagggc cctgtgatac                                                    20


<210>  66
<211>  20
<212>  DNA
<213>  Artificial Sequence

<220>
<223>  None

<400>  66
ctgcatgtgg ccggtatcac                                                    20


<210>  67
<211>  20
<212>  DNA
<213>  Artificial Sequence

<220>
<223>  None

<400>  67
tgcatgtggc cggtatcaca                                                    20


<210>  68
<211>  20
<212>  DNA

<213>  Artificial Sequence

<220>
<223>  None

<400>  68
tggccggtat cacagggccc                                                   20


<210>  69
<211>  20
<212>  DNA
<213>  Artificial Sequence

<220>
<223>  None

<400>  69
ggccggtatc acagggccct                                                   20


<210>  70
<211>  20
<212>  DNA
<213>  Artificial Sequence

<220>
<223>  None

<400>  70
cgcctgcctc agtttaccca                                                   20


<210>  71
<211>  20
<212>  DNA
<213>  Artificial Sequence

<220>
<223>  None

<400>  71
tcgcctgcct cagtttaccc                                                   20


<210>  72
<211>  20
<212>  DNA
<213>  Artificial Sequence

<220>
<223>  None

<400>  72
acagggccct gggtaaactg                                                   20


<210>  73
<211>  20
<212>  DNA
<213>  Artificial Sequence

<220>

<223>  None

<400>  73
ggccctgggt aaactgaggc                                                   20


<210>  74
<211>  20
<212>  DNA
<213>  Artificial Sequence

<220>
<223>  None

<400>  74
gcaggcgaca cagctgcatg                                                   20


<210>  75
<211>  20
<212>  DNA
<213>  Artificial Sequence

<220>
<223>  None

<400>  75
gcgacacagc tgcatgtggc                                                   20


<210>  76
<211>  20
<212>  DNA
<213>  Artificial Sequence

<220>
<223>  None

<400>  76
tacccagggc cctgtgatac                                                   20


<210>  77
<211>  20
<212>  DNA
<213>  Artificial Sequence

<220>
<223>  None

<400>  77
ctgcatgtgg ccggtatcac                                                   20


<210>  78
<211>  20
<212>  DNA
<213>  Artificial Sequence

<220>
<223>  None

<400>  78

tgcatgtggc cggtatcaca                                                    20

<210>  79
<211>  20
<212>  DNA
<213>  Artificial Sequence

<220>
<223>  None

<400>  79
tggccggtat cacagggccc                                                    20

<210>  80
<211>  20
<212>  DNA
<213>  Artificial Sequence

<220>
<223>  None

<400>  80
ggccggtatc acagggccct                                                    20

<210>  81
<211>  20
<212>  DNA
<213>  Artificial Sequence

<220>
<223>  None

<400>  81
cgcctgcctc agtttaccca                                                    20

<210>  82
<211>  20
<212>  DNA
<213>  Artificial Sequence

<220>
<223>  None

<400>  82
tcgcctgcct cagtttaccc                                                    20

<210>  83
<211>  20
<212>  DNA
<213>  Artificial Sequence

<220>
<223>  None

<400>  83
acagggccct gggtaaactg                                                    20

<210>  84
<211>  20
<212>  DNA
<213>  Artificial Sequence

<220>
<223>  None

<400>  84
ggccctgggt aaactgaggc                                                   20


<210>  85
<211>  20
<212>  DNA
<213>  Artificial Sequence

<220>
<223>  None

<400>  85
gcaggcgaca cagctgcatg                                                   20


<210>  86
<211>  20
<212>  DNA
<213>  Artificial Sequence

<220>
<223>  None

<400>  86
gcgacacagc tgcatgtggc                                                   20


<210>  87
<211>  20
<212>  DNA
<213>  Artificial Sequence

<220>
<223>  None

<400>  87
tacccagggc cctgtgatac                                                   20


<210>  88
<211>  20
<212>  DNA
<213>  Artificial Sequence

<220>
<223>  None

<400>  88
ctgcatgtgg ccggtatcac                                                   20


<210>  89
<211>  20
<212>  DNA

<213>  Artificial Sequence

<220>
<223>  None

<400>  89
tgcatgtggc cggtatcaca                                                   20


<210>  90
<211>  20
<212>  DNA
<213>  Artificial Sequence

<220>
<223>  None

<400>  90
tggccggtat cacagggccc                                                   20


<210>  91
<211>  20
<212>  DNA
<213>  Artificial Sequence

<220>
<223>  None

<400>  91
ggccggtatc acagggccct                                                   20


<210>  92
<211>  20
<212>  DNA
<213>  Artificial Sequence

<220>
<223>  None

<400>  92
cgcctgcctc agtttaccca                                                   20


<210>  93
<211>  20
<212>  DNA
<213>  Artificial Sequence

<220>
<223>  None

<400>  93
tcgcctgcct cagtttaccc                                                   20


<210>  94
<211>  20
<212>  DNA
<213>  Artificial Sequence

<220>

<223>  None

<400>  94
acagggccct gggtaaactg                                                   20


<210>  95
<211>  20
<212>  DNA
<213>  Artificial Sequence

<220>
<223>  None

<400>  95
ggccctgggt aaactgaggc                                                   20


<210>  96
<211>  20
<212>  DNA
<213>  Artificial Sequence

<220>
<223>  None

<400>  96
gcaggcgaca cagctgcatg                                                   20


<210>  97
<211>  20
<212>  DNA
<213>  Artificial Sequence

<220>
<223>  None

<400>  97
gctgcatgtg gccgtatcac                                                   20


<210>  98
<211>  20
<212>  DNA
<213>  Artificial Sequence

<220>
<223>  None

<400>  98
ttacccaggg ccctgtgata                                                   20


<210>  99
<211>  20
<212>  DNA
<213>  Artificial Sequence

<220>
<223>  None

<400>  99

ctgcatgtgg ccgtatcaca                                                    20

<210>  100
<211>  20
<212>  DNA
<213>  Artificial Sequence

<220>
<223>  None

<400>  100
gtggccgtat cacagggccc                                                    20

<210>  101
<211>  20
<212>  DNA
<213>  Artificial Sequence

<220>
<223>  None

<400>  101
tggccgtatc acagggccct                                                    20

<210>  102
<211>  20
<212>  DNA
<213>  Artificial Sequence

<220>
<223>  None

<400>  102
cacctgcctc agtttaccca                                                    20

<210>  103
<211>  20
<212>  DNA
<213>  Artificial Sequence

<220>
<223>  None

<400>  103
tcacctgcct cagtttaccc                                                    20

<210>  104
<211>  20
<212>  DNA
<213>  Artificial Sequence

<220>
<223>  None

<400>  104
acagggccct gggtaaactg                                                    20

<210>  105
<211>  20
<212>  DNA
<213>  Artificial Sequence

<220>
<223>  None

<400>  105
ggccctgggt aaactgaggc                                                    20


<210>  106
<211>  20
<212>  DNA
<213>  Artificial Sequence

<220>
<223>  None

<400>  106
gcaggtgaca cagctgcatg                                                    20


<210>  107
<211>  20
<212>  DNA
<213>  Artificial Sequence

<220>
<223>  None

<400>  107
gtgacacagc tgcatgtggc                                                    20


<210>  108
<211>  20
<212>  DNA
<213>  Artificial Sequence

<220>
<223>  None

<400>  108
gctgcatgtg gccggtatca                                                    20


<210>  109
<211>  20
<212>  DNA
<213>  Artificial Sequence

<220>
<223>  None

<400>  109
tatccagggc cccgtgatac                                                    20


<210>  110
<211>  20
<212>  DNA

<213>  Artificial Sequence

<220>
<223>  None

<400>  110
ctgcatgtgg ccggtatcac                                                   20


<210>  111
<211>  20
<212>  DNA
<213>  Artificial Sequence

<220>
<223>  None

<400>  111
tgcatgtggc cggtatcacg                                                   20


<210>  112
<211>  20
<212>  DNA
<213>  Artificial Sequence

<220>
<223>  None

<400>  112
tggccggtat cacggggccc                                                   20


<210>  113
<211>  20
<212>  DNA
<213>  Artificial Sequence

<220>
<223>  None

<400>  113
cgcctgcctc tgtttatcca                                                   20


<210>  114
<211>  20
<212>  DNA
<213>  Artificial Sequence

<220>
<223>  None

<400>  114
tcgcctgcct ctgtttatcc                                                   20


<210>  115
<211>  20
<212>  DNA
<213>  Artificial Sequence

<220>

<223>  None

<400>  115
acggggccct ggataaacag                                                   20


<210>  116
<211>  20
<212>  DNA
<213>  Artificial Sequence

<220>
<223>  None

<400>  116
ggccctggat aaacagaggc                                                   20


<210>  117
<211>  20
<212>  DNA
<213>  Artificial Sequence

<220>
<223>  None

<400>  117
gcaggcgaca cagctgcatg                                                   20


<210>  118
<211>  20
<212>  DNA
<213>  Artificial Sequence

<220>
<223>  None

<400>  118
gcgacacagc tgcatgtggc                                                   20


<210>  119
<211>  20
<212>  DNA
<213>  Artificial Sequence

<220>
<223>  None

<400>  119
gctgcatgtg gccggtatca                                                   20


<210>  120
<211>  20
<212>  DNA
<213>  Artificial Sequence

<220>
<223>  None

<400>  120

tacccagggc cccgtgatac                                                    20

<210>  121
<211>  20
<212>  DNA
<213>  Artificial Sequence

<220>
<223>  None

<400>  121
ctgcatgtgg ccggtatcac                                                    20

<210>  122
<211>  20
<212>  DNA
<213>  Artificial Sequence

<220>
<223>  None

<400>  122
tgcatgtggc cggtatcacg                                                    20

<210>  123
<211>  20
<212>  DNA
<213>  Artificial Sequence

<220>
<223>  None

<400>  123
tggccggtat cacggggccc                                                    20

<210>  124
<211>  20
<212>  DNA
<213>  Artificial Sequence

<220>
<223>  None

<400>  124
ggccggtatc acggggccct                                                    20

<210>  125
<211>  20
<212>  DNA
<213>  Artificial Sequence

<220>
<223>  None

<400>  125
cgcctgcctc agtttaccca                                                    20

<210>  126
<211>  20
<212>  DNA
<213>  Artificial Sequence

<220>
<223>  None

<400>  126
tcgcctgcct cagtttaccc                                                    20


<210>  127
<211>  20
<212>  DNA
<213>  Artificial Sequence

<220>
<223>  None

<400>  127
acggggccct gggtaaactg                                                    20


<210>  128
<211>  20
<212>  DNA
<213>  Artificial Sequence

<220>
<223>  None

<400>  128
ggccctgggt aaactgaggc                                                    20


<210>  129
<211>  20
<212>  DNA
<213>  Artificial Sequence

<220>
<223>  None

<400>  129
tgggtaaact gaggcaggcg                                                    20


<210>  130
<211>  20
<212>  DNA
<213>  Artificial Sequence

<220>
<223>  None

<400>  130
cctgagggac ttgatggggg                                                    20


<210>  131
<211>  20
<212>  DNA

<213>  Artificial Sequence

<220>
<223>  None

<400>  131
agacctgagg gacttgatgg                                                   20


<210>  132
<211>  20
<212>  DNA
<213>  Artificial Sequence

<220>
<223>  None

<400>  132
tagacctgag ggacttgatg                                                   20


<210>  133
<211>  20
<212>  DNA
<213>  Artificial Sequence

<220>
<223>  None

<400>  133
ctagacctga gggacttgat                                                   20


<210>  134
<211>  20
<212>  DNA
<213>  Artificial Sequence

<220>
<223>  None

<400>  134
cctagacctg agggacttga                                                   20


<210>  135
<211>  20
<212>  DNA
<213>  Artificial Sequence

<220>
<223>  None

<400>  135
ccacccccat caagtccctc                                                   20


<210>  136
<211>  20
<212>  DNA
<213>  Artificial Sequence

<220>

<223>  None

<400>  136
cctgccaaac ctagacctga                                                   20


<210>  137
<211>  20
<212>  DNA
<213>  Artificial Sequence

<220>
<223>  None

<400>  137
acctgccaaa cctagacctg                                                   20


<210>  138
<211>  20
<212>  DNA
<213>  Artificial Sequence

<220>
<223>  None

<400>  138
ccatcaagtc cctcaggtct                                                   20


<210>  139
<211>  20
<212>  DNA
<213>  Artificial Sequence

<220>
<223>  None

<400>  139
aagtccctca ggtctaggtt                                                   20


<210>  140
<211>  20
<212>  DNA
<213>  Artificial Sequence

<220>
<223>  None

<400>  140
ccctcaggtc taggtttggc                                                   20


<210>  141
<211>  20
<212>  DNA
<213>  Artificial Sequence

<220>
<223>  None

<400>  141

aggtctaggt ttggcaggtt                                                    20

<210>  142
<211>  20
<212>  DNA
<213>  Artificial Sequence

<220>
<223>  None

<400>  142
ggcaaaaaca cagcaacgct                                                    20

<210>  143
<211>  20
<212>  DNA
<213>  Artificial Sequence

<220>
<223>  None

<400>  143
gctcggttaa atctgaattt                                                    20

<210>  144
<211>  20
<212>  DNA
<213>  Artificial Sequence

<220>
<223>  None

<400>  144
ctcggttaaa tctgaatttc                                                    20

<210>  145
<211>  20
<212>  DNA
<213>  Artificial Sequence

<220>
<223>  None

<400>  145
aatttcgggt aagtatatcc                                                    20

<210>  146
<211>  20
<212>  DNA
<213>  Artificial Sequence

<220>
<223>  None

<400>  146
atttcgggta agtatatcct                                                    20

<210>  147
<211>  20
<212>  DNA
<213>  Artificial Sequence

<220>
<223>  None

<400>  147
gtctcttcca aatgaggccc                                                   20


<210>  148
<211>  20
<212>  DNA
<213>  Artificial Sequence

<220>
<223>  None

<400>  148
agtatatcct gggcctcatt                                                   20


<210>  149
<211>  20
<212>  DNA
<213>  Artificial Sequence

<220>
<223>  None

<400>  149
atctaagtct cttccaaatg                                                   20


<210>  150
<211>  20
<212>  DNA
<213>  Artificial Sequence

<220>
<223>  None

<400>  150
aaaaaacgtc gagaccagcc                                                   20


<210>  151
<211>  20
<212>  DNA
<213>  Artificial Sequence

<220>
<223>  None

<400>  151
tttcaccgtg ttggccgggc                                                   20


<210>  152
<211>  20
<212>  DNA

<213>  Artificial Sequence

<220>
<223>  None

<400>  152
ggggtttcac cgtgttggcc                                                   20


<210>  153
<211>  20
<212>  DNA
<213>  Artificial Sequence

<220>
<223>  None

<400>  153
cggggtttca ccgtgttggc                                                   20


<210>  154
<211>  20
<212>  DNA
<213>  Artificial Sequence

<220>
<223>  None

<400>  154
cgagaccagc ccggccaaca                                                   20


<210>  155
<211>  20
<212>  DNA
<213>  Artificial Sequence

<220>
<223>  None

<400>  155
gagacggggt ttcaccgtgt                                                   20


<210>  156
<211>  20
<212>  DNA
<213>  Artificial Sequence

<220>
<223>  None

<400>  156
ttgtattttt agtagagacg                                                   20


<210>  157
<211>  20
<212>  DNA
<213>  Artificial Sequence

<220>

<223>  None

<400>  157
tttgtatttt tagtagagac                                                   20


<210>  158
<211>  20
<212>  DNA
<213>  Artificial Sequence

<220>
<223>  None

<400>  158
ttttgtattt ttagtagaga                                                   20


<210>  159
<211>  20
<212>  DNA
<213>  Artificial Sequence

<220>
<223>  None

<400>  159
aaaaatacaa aaaattagcc                                                   20


<210>  160
<211>  20
<212>  DNA
<213>  Artificial Sequence

<220>
<223>  None

<400>  160
caggcgtgag ccactgcgcc                                                   20


<210>  161
<211>  20
<212>  DNA
<213>  Artificial Sequence

<220>
<223>  None

<400>  161
aaaaaattag ccaggcgcag                                                   20


<210>  162
<211>  20
<212>  DNA
<213>  Artificial Sequence

<220>
<223>  None

<400>  162

tcccagagtg ctgggatcac                                                    20

<210>  163
<211>  20
<212>  DNA
<213>  Artificial Sequence

<220>
<223>  None

<400>  163
cctcagcctc ccagagtgct                                                    20

<210>  164
<211>  20
<212>  DNA
<213>  Artificial Sequence

<220>
<223>  None

<400>  164
cgcctgtgat cccagcactc                                                    20

<210>  165
<211>  20
<212>  DNA
<213>  Artificial Sequence

<220>
<223>  None

<400>  165
gcctcagcct cccagagtgc                                                    20

<210>  166
<211>  20
<212>  DNA
<213>  Artificial Sequence

<220>
<223>  None

<400>  166
gcctgtgatc ccagcactct                                                    20

<210>  167
<211>  20
<212>  DNA
<213>  Artificial Sequence

<220>
<223>  None

<400>  167
tgtgatccca gcactctggg                                                    20

<210>  168
<211>  20
<212>  DNA
<213>  Artificial Sequence

<220>
<223>  None

<400>  168
cccagcactc tgggaggctg                                                   20


<210>  169
<211>  20
<212>  DNA
<213>  Artificial Sequence

<220>
<223>  None

<400>  169
gcactctggg aggctgaggc                                                   20


<210>  170
<211>  20
<212>  DNA
<213>  Artificial Sequence

<220>
<223>  None

<400>  170
ctctgggagg ctgaggcagg                                                   20


<210>  171
<211>  20
<212>  DNA
<213>  Artificial Sequence

<220>
<223>  None

<400>  171
tgaggcaggc ggatcacccg                                                   20


<210>  172
<211>  20
<212>  DNA
<213>  Artificial Sequence

<220>
<223>  None

<400>  172
ggtcttgaac atctgacctc                                                   20


<210>  173
<211>  20
<212>  DNA

<213>  Artificial Sequence

<220>
<223>  None

<400>  173
tggtcttgaa catctgacct                                                    20


<210>  174
<211>  20
<212>  DNA
<213>  Artificial Sequence

<220>
<223>  None

<400>  174
tcagatgttc aagaccagcc                                                    20


<210>  175
<211>  20
<212>  DNA
<213>  Artificial Sequence

<220>
<223>  None

<400>  175
tttcgccctg tcggccaggc                                                    20


<210>  176
<211>  20
<212>  DNA
<213>  Artificial Sequence

<220>
<223>  None

<400>  176
agtgtttcgc cctgtcggcc                                                    20


<210>  177
<211>  20
<212>  DNA
<213>  Artificial Sequence

<220>
<223>  None

<400>  177
tcaagaccag cctggccgac                                                    20


<210>  178
<211>  20
<212>  DNA
<213>  Artificial Sequence

<220>

<223>  None

<400>  178
caagaccagc ctggccgaca                                                   20


<210>  179
<211>  20
<212>  DNA
<213>  Artificial Sequence

<220>
<223>  None

<400>  179
gagacagtgt ttcgccctgt                                                   20


<210>  180
<211>  20
<212>  DNA
<213>  Artificial Sequence

<220>
<223>  None

<400>  180
ctacaaatac aaaaattagc                                                   20


<210>  181
<211>  20
<212>  DNA
<213>  Artificial Sequence

<220>
<223>  None

<400>  181
tacaaataca aaaattagcc                                                   20


<210>  182
<211>  20
<212>  DNA
<213>  Artificial Sequence

<220>
<223>  None

<400>  182
atacaaaaat tagccgggag                                                   20


<210>  183
<211>  20
<212>  DNA
<213>  Artificial Sequence

<220>
<223>  None

<400>  183

caggcacctg ccaccactcc                                                    20


<210>  184
<211>  20
<212>  DNA
<213>  Artificial Sequence

<220>
<223>  None

<400>  184
caaaaattag ccgggagtgg                                                    20


<210>  185
<211>  20
<212>  DNA
<213>  Artificial Sequence

<220>
<223>  None

<400>  185
aattagccgg gagtggtggc                                                    20


<210>  186
<211>  20
<212>  DNA
<213>  Artificial Sequence

<220>
<223>  None

<400>  186
tcctgaatag ctgagattac                                                    20


<210>  187
<211>  20
<212>  DNA
<213>  Artificial Sequence

<220>
<223>  None

<400>  187
gcctgtaatc tcagctattc                                                    20


<210>  188
<211>  20
<212>  DNA
<213>  Artificial Sequence

<220>
<223>  None

<400>  188
tgtaatctca gctattcagg                                                    20

<210>  189
<211>  20
<212>  DNA
<213>  Artificial Sequence

<220>
<223>  None

<400>  189
ctcagctatt caggaggctg                                                    20


<210>  190
<211>  20
<212>  DNA
<213>  Artificial Sequence

<220>
<223>  None

<400>  190
gctattcagg aggctgaggc                                                    20


<210>  191
<211>  20
<212>  DNA
<213>  Artificial Sequence

<220>
<223>  None

<400>  191
gcaggagaat cacttgaacc                                                    20


<210>  192
<211>  20
<212>  DNA
<213>  Artificial Sequence

<220>
<223>  None

<400>  192
caggagaatc acttgaacct                                                    20


<210>  193
<211>  20
<212>  DNA
<213>  Artificial Sequence

<220>
<223>  None

<400>  193
gagaatcact tgaacctggg                                                    20


<210>  194
<211>  20
<212>  DNA

<213>  Artificial Sequence

<220>
<223>  None

<400>  194
aatcacttga acctgggagg                                                    20


<210>  195
<211>  20
<212>  DNA
<213>  Artificial Sequence

<220>
<223>  None

<400>  195
cacggcaacc tccgcctccc                                                    20


<210>  196
<211>  20
<212>  DNA
<213>  Artificial Sequence

<220>
<223>  None

<400>  196
cacttgaacc tgggaggcgg                                                    20


<210>  197
<211>  20
<212>  DNA
<213>  Artificial Sequence

<220>
<223>  None

<400>  197
aggcggaggt tgccgtgagc                                                    20


<210>  198
<211>  20
<212>  DNA
<213>  Artificial Sequence

<220>
<223>  None

<400>  198
ggcggaggtt gccgtgagcc                                                    20


<210>  199
<211>  20
<212>  DNA
<213>  Artificial Sequence

<220>

<223>  None

<400>  199
ggtggcgtga tcccggctca                                                   20


<210>  200
<211>  20
<212>  DNA
<213>  Artificial Sequence

<220>
<223>  None

<400>  200
ggagtgcggt ggcgtgatcc                                                   20


<210>  201
<211>  20
<212>  DNA
<213>  Artificial Sequence

<220>
<223>  None

<400>  201
tcgcccaggc tggagtgcgg                                                   20


<210>  202
<211>  20
<212>  DNA
<213>  Artificial Sequence

<220>
<223>  None

<400>  202
ctatcgccca ggctggagtg                                                   20


<210>  203
<211>  20
<212>  DNA
<213>  Artificial Sequence

<220>
<223>  None

<400>  203
cacgccaccg cactccagcc                                                   20


<210>  204
<211>  20
<212>  DNA
<213>  Artificial Sequence

<220>
<223>  None

<400>  204

acgccaccgc actccagcct                                                    20

<210>  205
<211>  20
<212>  DNA
<213>  Artificial Sequence

<220>
<223>  None

<400>  205
tcttgctcta tcgcccaggc                                                    20

<210>  206
<211>  20
<212>  DNA
<213>  Artificial Sequence

<220>
<223>  None

<400>  206
agagtcttgc tctatcgccc                                                    20

<210>  207
<211>  20
<212>  DNA
<213>  Artificial Sequence

<220>
<223>  None

<400>  207
   ggttttttaa tttatttttt                                                      20

<210>  208
<211>  20
<212>  DNA
<213>  Artificial Sequence

<220>
<223>  None

<400>  208
aaatgtcaga taatcaatgt                                                    20

<210>  209
<211>  20
<212>  DNA
<213>  Artificial Sequence

<220>
<223>  None

<400>  209
caaatgtcag ataatcaatg                                                    20

<210>  210
<211>  20
<212>  DNA
<213>  Artificial Sequence

<220>
<223>  None

<400>  210
ggggcctcca gacaaaattc                                                    20


<210>  211
<211>  20
<212>  DNA
<213>  Artificial Sequence

<220>
<223>  None

<400>  211
tgtgcatcct gaattttgtc                                                    20


<210>  212
<211>  20
<212>  DNA
<213>  Artificial Sequence

<220>
<223>  None

<400>  212
gcatcctgaa ttttgtctgg                                                    20


<210>  213
<211>  20
<212>  DNA
<213>  Artificial Sequence

<220>
<223>  None

<400>  213
acgctggatt ggctcgggtg                                                    20


<210>  214
<211>  20
<212>  DNA
<213>  Artificial Sequence

<220>
<223>  None

<400>  214
gacgctggat tggctcgggt                                                    20


<210>  215
<211>  20
<212>  DNA

<213>  Artificial Sequence

<220>
<223>  None

<400>  215
agacgctgga ttggctcggg                                                    20


<210>  216
<211>  20
<212>  DNA
<213>  Artificial Sequence

<220>
<223>  None

<400>  216
acaagacgct ggattggctc                                                    20


<210>  217
<211>  20
<212>  DNA
<213>  Artificial Sequence

<220>
<223>  None

<400>  217
gacaagacgc tggattggct                                                    20


<210>  218
<211>  20
<212>  DNA
<213>  Artificial Sequence

<220>
<223>  None

<400>  218
agggggacaa gacgctggat                                                    20


<210>  219
<211>  20
<212>  DNA
<213>  Artificial Sequence

<220>
<223>  None

<400>  219
ggagaagggg gacaagacgc                                                    20


<210>  220
<211>  20
<212>  DNA
<213>  Artificial Sequence

<220>

<223>  None

<400>  220
tgatgaaaag ggggagaagg                                                   20


<210>  221
<211>  20
<212>  DNA
<213>  Artificial Sequence

<220>
<223>  None

<400>  221
ttgatgaaaa gggggagaag                                                   20


<210>  222
<211>  20
<212>  DNA
<213>  Artificial Sequence

<220>
<223>  None

<400>  222
gttgatgaaa agggggagaa                                                   20


<210>  223
<211>  20
<212>  DNA
<213>  Artificial Sequence

<220>
<223>  None

<400>  223
cgttgatgaa aagggggaga                                                   20


<210>  224
<211>  20
<212>  DNA
<213>  Artificial Sequence

<220>
<223>  None

<400>  224
acagggcgtt gatgaaaagg                                                   20


<210>  225
<211>  20
<212>  DNA
<213>  Artificial Sequence

<220>
<223>  None

<400>  225

cacagggcgt tgatgaaaag                                                    20

<210>  226
<211>  20
<212>  DNA
<213>  Artificial Sequence

<220>
<223>  None

<400>  226
gcacagggcg ttgatgaaaa                                                    20

<210>  227
<211>  20
<212>  DNA
<213>  Artificial Sequence

<220>
<223>  None

<400>  227
ggcacagggc gttgatgaaa                                                    20

<210>  228
<211>  20
<212>  DNA
<213>  Artificial Sequence

<220>
<223>  None

<400>  228
tttcatcaac gccctgtgcc                                                    20

<210>  229
<211>  20
<212>  DNA
<213>  Artificial Sequence

<220>
<223>  None

<400>  229
acttcctctc ccctggcaca                                                    20

<210>  230
<211>  20
<212>  DNA
<213>  Artificial Sequence

<220>
<223>  None

<400>  230
ttcatcaacg ccctgtgcca                                                    20

<210>  231
<211>  20
<212>  DNA
<213>  Artificial Sequence

<220>
<223>  None

<400>  231
cacttcctct cccctggcac                                                   20


<210>  232
<211>  20
<212>  DNA
<213>  Artificial Sequence

<220>
<223>  None

<400>  232
tcatcaacgc cctgtgccag                                                   20


<210>  233
<211>  20
<212>  DNA
<213>  Artificial Sequence

<220>
<223>  None

<400>  233
aacgccctgt gccaggggag                                                   20


<210>  234
<211>  20
<212>  DNA
<213>  Artificial Sequence

<220>
<223>  None

<400>  234
gccctccact tcctctcccc                                                   20


<210>  235
<211>  20
<212>  DNA
<213>  Artificial Sequence

<220>
<223>  None

<400>  235
ctgtgccagg ggagaggaag                                                   20


<210>  236
<211>  20
<212>  DNA

<213>  Artificial Sequence

<220>
<223>  None

<400>  236
tgccagggga gaggaagtgg                                                   20


<210>  237
<211>  20
<212>  DNA
<213>  Artificial Sequence

<220>
<223>  None

<400>  237
gccaggggag aggaagtgga                                                   20


<210>  238
<211>  20
<212>  DNA
<213>  Artificial Sequence

<220>
<223>  None

<400>  238
ggagaggaag tggagggcgc                                                   20


<210>  239
<211>  20
<212>  DNA
<213>  Artificial Sequence

<220>
<223>  None

<400>  239
aggaagtgga gggcgctggc                                                   20


<210>  240
<211>  20
<212>  DNA
<213>  Artificial Sequence

<220>
<223>  None

<400>  240
tggagggcgc tggccggccg                                                   20


<210>  241
<211>  20
<212>  DNA
<213>  Artificial Sequence

<220>

<223>  None

<400>  241
ggagggcgct ggccggccgt                                                   20


<210>  242
<211>  20
<212>  DNA
<213>  Artificial Sequence

<220>
<223>  None

<400>  242
gagggcgctg gccggccgtg                                                   20


<210>  243
<211>  20
<212>  DNA
<213>  Artificial Sequence

<220>
<223>  None

<400>  243
ccgttgcatt gccccacggc                                                   20


<210>  244
<211>  20
<212>  DNA
<213>  Artificial Sequence

<220>
<223>  None

<400>  244
ccggccgtgg ggcaatgcaa                                                   20


<210>  245
<211>  20
<212>  DNA
<213>  Artificial Sequence

<220>
<223>  None

<400>  245
tcttatagcc ctgtgctggg                                                   20


<210>  246
<211>  20
<212>  DNA
<213>  Artificial Sequence

<220>
<223>  None

<400>  246

atgcaacggc ctcccagcac                                                    20

<210>  247
<211>  20
<212>  DNA
<213>  Artificial Sequence

<220>
<223>  None

<400>  247
tgcaacggcc tcccagcaca                                                    20

<210>  248
<211>  20
<212>  DNA
<213>  Artificial Sequence

<220>
<223>  None

<400>  248
tcctcttata gccctgtgct                                                    20

<210>  249
<211>  20
<212>  DNA
<213>  Artificial Sequence

<220>
<223>  None

<400>  249
ctcctcttat agccctgtgc                                                    20

<210>  250
<211>  20
<212>  DNA
<213>  Artificial Sequence

<220>
<223>  None

<400>  250
tcccagcaca gggctataag                                                    20

<210>  251
<211>  20
<212>  DNA
<213>  Artificial Sequence

<220>
<223>  None

<400>  251
cacagggcta taagaggagc                                                    20

<210>  252
<211>  20
<212>  DNA
<213>  Artificial Sequence

<220>
<223>  None

<400>  252
acagggctat aagaggagcc                                                   20


<210>  253
<211>  20
<212>  DNA
<213>  Artificial Sequence

<220>
<223>  None

<400>  253
gtgtcwtcct cgcctgtgtc                                                   20


<210>  254
<211>  20
<212>  DNA
<213>  Artificial Sequence

<220>
<223>  None

<400>  254
cccacaatct ccgaggccag                                                   20


<210>  255
<211>  19
<212>  DNA
<213>  Artificial Sequence

<220>
<223>  None

<400>  255
ctgggctaca ctgagcacc                                                    19


<210>  256
<211>  21
<212>  DNA
<213>  Artificial Sequence

<220>
<223>  None

<400>  256
aagtggtcgt tgagggcaat g                                                 21


<210>  257
<211>  21
<212>  DNA

<213>  Artificial Sequence

<220>
<223>  None

<400>  257
catgtacgtt gctatccagg c                                                21


<210>  258
<211>  21
<212>  DNA
<213>  Artificial Sequence

<220>
<223>  None

<400>  258
ctccttaatg tcacgcacga t                                                21

## Description

The present invention is directed to a composition for the targeted knockout of a gene on double-stranded DNA in a biological cell, a method for the targeted knockout of a gene on double-stranded DNA in a biological cell, a preparation comprising a biological cell prepared *in vitro,* said biological cell comprises a gene on double-stranded DNA, which is knocked-out in a targeted manner, a kit for the targeted knockout of a gene on double-stranded DNA in a biological cell, a method of treating a subject afflicted with a disease associated with a mutated gene, nucleic acid molecules which can be a component of said composition and methods, and a method of treating a subject afflicted with a disease associated with a mutated gene.

### FIELD OF THE INVENTION

The present invention relates to the field of molecular medicine, more particularly to the field of genetic engineering applications, preferably to the targeted knockout of disease-associated genes.

### BACKGROUND OF THE INVENTION

A genetic disorder is a health problem caused by one or more abnormalities in the genome. It can be caused by a mutation in a single gene (monogenic) or multiple genes (polygenic) or by a chromosomal abnormality. Although polygenic disorders are the most common, the term is mostly used when discussing disorders with a single genetic cause, either in a gene or chromosome.

Two copies of the gene must be mutated for a person to be affected by an autosomal recessive disorder. An affected person usually has unaffected parents who each carry a single copy of the mutated gene and are referred to as genetic carriers. Each parent with a defective gene normally do not have symptoms. Two unaffected people who each carry one copy of the mutated gene have a 25% risk with each pregnancy of having a child affected by the disorder.

Examples of autosomal recessive disorders are albinism, medium-chain acyl-CoA dehydrogenase deficiency, cystic fibrosis, sickle cell disease, Tay-Sachs disease, Niemann-Pick disease, spinal muscular atrophy, and Roberts syndrome.

Only one mutated copy of the gene will be necessary for a person to be affected by an autosomal dominant disorder. Each affected person usually has one affected parent. The chance a child will inherit the mutated gene is 50%. Autosomal dominant conditions sometimes have reduced penetrance, which means although only one mutated copy is needed, not all individuals who inherit that mutation go on to develop the disease.

Examples of autosomal dominant disorders are Huntington's disease, neurofibromatosis type 1, neurofibromatosis type 2, Marfan syndrome, hereditary nonpolyposis colorectal cancer, hereditary multiple exostoses (a highly penetrant autosomal dominant disorder), tuberous sclerosis, Von Willebrand disease, and acute intermittent porphyria. Birth defects are also called congenital anomalies.

Neutropenia is an abnormally low concentration of neutrophils in the blood. Neutrophils make up the majority of circulating white blood cells and serve as the primary defense against infections by destroying bacteria, bacterial fragments and immunoglobulin-bound viruses in the blood. People with neutropenia are more susceptible to bacterial infections and, without prompt medical attention, the condition may become life-threatening. Neutropenia can be divided into congenital and acquired, with severe congenital neutropenia and cyclic neutropenia.

Severe congenital neutropenia (SCN or CN), also often known as Kostmann syndrome or disease, is an inherited bone marrow failure syndrome. CN affects myelopoiesis, usually without other physical malformations.

Autosomal-dominant mutations in the so-called "elastase neutrophil expressed gene" (*ELANE*) are the most common cause of CN (*ELANE*-CN). "Maturation arrest", the failure of the bone marrow myeloid progenitors to form mature neutrophils, is a consistent feature of *ELANE-*CN*.* Mutated neutrophil elastase could not be properly processed, accumulates in the cells, inducing unfolded protein response (UPR) and endoplasmic reticulum (ER) stress. This leads to the death of hematopoietic cells and defective granulopoiesis. *ELANE-*CN manifests in infancy with life-threatening bacterial infections.

Another congenital bone marrow failure syndrome which is caused by ELANE mutations is cyclic neutropenia (ELANE-CyN). In CyN patients the numbers of mature neutrophils show cycling from zero to almost normal levels (under G-CSF treatment) in approximately 21-days intervals with a high probability of severe infections during the neutropenia phase.

Daily administration of granulocyte colony stimulating factor (G-CSF), mostly in recombinant form or the human variant (rhG-CSF; filgrastim), to patients suffering from *ELANE-*CN and *ELANE*-CyN in many cases clinically improves neutrophil counts and immune function. G-CSF stimulates the production of more neutrophils and delays their apoptosis. It often decreases the severity and frequency of infections and, therefore, is the mainstay of therapy. Overall survival is now estimated to exceed 80% although 10% of the patients still die from severe bacterial infections or sepsis.

This form of G-CSF therapy of *ELANE-*CN and *ELANE*-CyN may, however, increase the risk for long term side effects. Approximately 20 % of the patients develop myelodysplasia or acute myeloid leukemia. The most common leukemia in CN and CyN is AML, but acute lymphoid leukemia (ALL), juvenile myelomonocytic leukemia (JMML), chronic myelomonocytic leukemia (CMML), and bi- phenotypic leukemia are also reported in the literature. It was previously demonstrated that patients who had a robust response to G-CSF (doses<8 pg/kg/day) had a cumulative incidence of 15% for developing MDS/leukemia after 15 years on G-CSF, while an incidence of 34% was reported in patients with poor response to G-CSF despite high doses. In addition, approximately 15 % of CN and CyN patients do not respond to G-CSF even at doses of up to 50 µg/kg/day.

Hematopoietic stem cell transplant (HSCT) is an alternative, curative therapy for patients who do not respond to G-CSF therapy or who develop AML/MDS. However, patients with chronic neutropenia who undergo HCT are at increased risk of developing infectious complications such as fungal and graft-versus-host disease. Moreover, HCT requires a matched related donor for successful survival but most patients will not have an available matched donor.

WO 2019/217294 discloses a method for inactivating a mutant allele of the ELANE gene associated with CN and CyN. The known method involves the introduction to a biological cell of a CRISPR nuclease or a sequence encoding the CRISPR nuclease together with a guide RNA molecule. The complex of the CRISPR nuclease and the RNA molecule is described to affect a double strand break in the mutant allele of the *ELANE* gene. This method is complex and cumbersome and has to be adapted to each patient individually. It depends on the existence of single nucleotide polymorphisms (SNPs) which, however, cannot be found or used in all patients in the same manner.

Nasri et al. (2020), CRISPR/Cas9-mediated ELANE knockout enables neutrophilic maturation of primary hematopoietic stem and progenitor cells and induced pluripotent stem cells of severe congenital neutropenia patients, Haematologica 105(3), pp. 598-609, disclose a similar method. In primary hematopoietic stem and progenitor cells from *ELANE-*CN and *ELANE*-CyN patients the mutated *ELANE* gene is knocked-out by causing DNA double-strand breaks by introducing a CRISPR/Cas9-sgRNA ribonucleo-protein.

However, the approaches disclosed in WO 2019/27294 and Nasri et al. may result in the introduction of novel *ELANE* mutations and malignant transformations of the treated cells and, for this reason, may be inappropriate for an application in clinical practices.

Against this background it is an object underlying the invention to provide an improved method and composition for the targeted knockout of a gene on double-stranded DNA in a biological cell, by means of which the disadvantages of the methods known in the art can be avoided or at least reduced. In, particular a method and composition should be provided allowing the treatment of an autosomal-dominantly inherited genetic disorder, such as congenital neutropenia (CN) or cyclic neutropenia (CyN), in particular *ELANE-*CN and *ELANE*-CyN, in a reliable and safe manner.

The present invention satisfies these and other needs.

### SUMMARY OF THE INVENTION

The present invention provides a composition for the targeted knockout of a gene on double-stranded DNA in a biological cell, comprising:
- a first CRISPR endonuclease or a nucleic acid molecule encoding the said CRISPR endonuclease;
- a second CRISPR endonuclease or a nucleic acid molecule encoding said second CRISPR endonuclease;
   said first and said second CRISPR endonucleases are configured to create single-strand DNA breaks,
- a first single guide RNA (sgRNA), and
- a second sgRNA,
   said first sgRNA is configured to hybridize to the sense strand of a genetic element controlling the expression of said gene, and
   said second sgRNA is configured to hybridize to the antisense strand of said genetic element controlling the expression of said gene.

According to the invention, a "targeted knockout" or "gene knockout" refers to a specific functional elimination of the expression of a gene on double stranded DNA by a genetic technique, however without destructing and/or affecting the coding region of said gene.

According to the invention "double-stranded DNA" (dsDNA) refers to a molecule of DNA consisting of two parallel polynucleotide chains joined by hydrogen bonds between complementary purines and pyrimidines.

According to the invention a "biological cell" includes both prokaryotic and eukaryotic cells, such as animal, plant or bacterial cells comprising DNA, in particular dsDNA. Human cells comprising dsDNA are of particular preference.

According to the invention, a "CRIPR endonuclease" refers to an enzyme which cleaves the phosphodiester bond within a polynucleotide chain and which is a component of a CRISPR (Clustered Regularly Interspaced Short Palindromic Repeat) complex. The CRISPR endonuclease is, therefore, capable to specifically bind to a guide or sgRNA which directs the endonuclease to a target nucleic acid. A CRISPR endonuclease is also referred to as 'CRISPR associated protein' (Cas) or CRISPR associated endonuclease, respectively.

According to the invention, in a first embodiment the first and second CRISPR endonucleases are identical, and in a second embodiment the first and second CRISPR endonucleases differ from each other.

According to the invention "a nucleic acid molecule encoding the CRISPR endonuclease" refers to a DNA, or cDNA or mRNA, which comprise or consist of the coding information for the synthesis of the CRISPR endonuclease.

According to the invention the first and second CRISPR endonucleases are configured to create single-strand DNA breaks on the target nucleic acid, but no double-strand DNA breaks, as typically carried out by naturally occurring CRISPR endonucleases, such as the types I, II, and IIIa. Such a configuration of the CRISPR endonucleases to be used in the invention can be achieved by a targeted modification of wild type CRISPR endonucleases. The modification typically addresses the catalytic center of the enzyme, thereby converting the nuclease into a nicking enzyme or "nickase", respectively. As a consequence the modified CRISPR endonuclease according to the invention cuts one strand of a double-stranded DNA at a specific recognition nucleotide sequences known as a restriction site but is no longer capable to introduce DNA double-strand breaks. It hydrolyses (cuts) only one strand of the DNA duplex, to produce DNA molecules that are "nicked", rather than cleaved.

According to the invention a "single guide RNA" (sgRNA) or guide RNA is a component of the CRISPR complex which serves to guide the CRISPR endonuclease to its target. The sgRNA is a non-coding short RNA sequence which binds to the complementary target DNA sequence. The sgRNA first binds to the CRISPR endonuclease enzyme and the sgRNA sequence guides the complex via pairing to a specific location on the DNA, where the modified CRISPR endonuclease performs its nickase activity by cutting the target single DNA strand.

According to the invention a "genetic element controlling the expression of said gene" refers to one or several regions located on the DNA bearing the target gene to be knocked-out, said region(s) control(s) the initiation of transcription of the gene. It is typically located near the transcription start of the gene, mostly upstream of the coding sequence. This region can be short (only a few nucleotides in length) or quite long (hundreds of nucleotides long). A typical genetic element controlling the expression of said gene according to the invention is a promoter.

According to the invention the first sgRNA comprises a nucleotide sequence which is essentially complementary to a sequence on the sense strand of the DNA bearing the coding sequence of said gene to be knocked-out. Said complementary sequence is located in a genetic element controlling the expression of said gene, typically upstream of the gene.

According to the invention the second sgRNA comprises a nucleotide sequence which is essentially complementary to a sequence on the antisense strand of the DNA bearing the coding sequence of said gene to be knocked-out. Said complementary sequence is located in a genetic element controlling the expression of said gene.

The essential complementary sequences of the first and second sgRNA allow the specific hybridization of the complex consisting of modified CRISPR endonuclease and sgRNA to the sense and antisense strand of the expression controlling element and the performance of a single-strand break thereof.

The inventors provide a genetic tool by means of which the targeted knockout of any gene on double-stranded DNA can be achieved in a reliable and safely manner. The safety profile of this approach is very high because the disruption of the controlling element, e.g. the promoter, occurs via the use of two CRISPR nickases with two sgRNAs. The nickase may be a mutated variant of a conventional CRISPR endonuclease, such as Cas9, that can only generated single-strand DNA breaks (SSB).

By using two guide RNAs or sgRNAs, respectively, as paired nickases, designed for the sense (plus) and antisense (minus) DNA strands, it is finally possible to create a double-strand break (DSB) by generating two single-strand breaks at the controlling element, e.g. the promoter upstream of the transcription start site of the gene to be knocked-out. The DSB would be created only if the two CRISPR endonucleases will cut simultaneously at the target sites. If any of the sgRNA will target any unwanted site in the genome and introduce a SSB non-specifically, there will be no adverse effects because the SSB will be repaired efficiently by the cellular repair machinery. Therefore, the composition according to the invention comprises one sgRNA that introduces a nick at the plus or sense strand of the controlling element upstream of the transcription start site (TSS) and one sgRNA that introduce the complementary nick at the minus or antisense strand upstream of the TSS. Simultaneous activity of both CRISPR endonulcease/sgRNA complexes lead to small genomic deletions in the controlling element, that permanently repress the gene transcription initiation complex.

The invention does not affect the coding sequence of the gene to be knocked-out in contrast to what is taught in the art, e.g. in Nasri et al. (2019, *I.c*.) or WO 2019/217294. The disruption of the coding sequence may result in malignant transformations. By using the invention the risk of malignant transformation is excluded or at least significantly reduced. Thus the composition of the invention is characterized by a very high safety profile which qualifies it for an administration in clinical routine applications.

The problem underlying the invention is herewith completely solved.

In an embodiment of the invention said genetic element controlling the expression of said gene is a promoter.

This measure has the advantage that such a controlling element is targeted which reliably and safely ensures the permanent knockout of the gene, however without affecting the coding region of the gene. According to the invention the promoter is a sequence of DNA to which proteins bind that initiate transcription of a single RNA from the DNA downstream of it. This transcribed RNA encodes the gene to be knocked-out. The promoter is typically located near the transcription start site of the gene, upstream on the DNA, i.e. towards the 5' region of the sense strand. Promoters can be about 100-1000 base pairs long.

In another embodiment of the invention said first and/or second CRISPR endonuclease is a variant of the CRISPR associated protein 9 (Cas9).

This measure has the advantage that such CRISPR endonuclease is used for a conversion into a nickase, which is well established and characterized in the art. Cas9, formerly also called Cas5, Csn1, or Csx12, is a 160 kilodalton protein. It originates from the Gram-positive, aerotolerant bacterium *Streptococcus pyogenes* where it plays a vital role in the immunological defense against DNA viruses and plasmids. *Streptococcus pyogenes* utilizes CRISPR to memorize and Cas9 to later interrogate and cleave foreign DNA, such as invading bacteriophage DNA or plasmid DNA. Cas9 performs this interrogation by unwinding foreign DNA and checking for sites complementary to the 20 basepair spacer region of the guide RNA. If the DNA substrate is complementary to the guide RNA, Cas9 cleaves the invading DNA. Apart from its original function in bacterial immunity, the Cas9 protein has been heavily utilized as a genome engineering tool to induce site-directed double-strand breaks in DNA. Cas9 can cleave nearly any sequence complementary to the guide RNA. Because the target specificity of Cas9 stems from the guide RNA:DNA complementarity and not modifications to the protein itself, engineering Cas9 to target new DNA is straightforward. Native Cas9 requires a guide RNA composed of two disparate RNAs that associate - the CRISPR RNA (crRNA), and the trans-activating crRNA (tracrRNA).Cas9 targeting has been simplified through the engineering of a chimeric single guide RNA (chiRNA or sgRNA).

In another embodiment of the invention said variant of Cas9 comprises mutation(s) in the nuclease domain(s) RuvC and/or HNH, said mutation(s) conferring DNA nickase activity.

This measure has the advantage that the nickase conversion of Cas9 is effectively and reliably achieved by specifically addressing those catalytic domains in Cas9 which are responsible for the DNA cleavage activity. The mutations in the RuvC and/or HNH domains eliminate the activity of Cas9 to carry out DNA double strand breaks but establish the activity to effect DNA single-strand breaks.

In another embodiment of the invention said variant of Cas 9 is Cas9 D10A nickase and/or Cas9 H840A nickase.

This measure has the advantage that CRISPR endonucleases are employed, which are well established and characterized by their nicking enzyme activities which makes them predestined for a use in the invention. By the D10A amino acid exchange the RuvC catalytic center is disrupted, and by the H840A amino acid exchange the HNH catalytic domain is disrupted, both converting the enzyme into a nicking enzyme.

In another embodiment of the invention said gene is a mutated form of a wild type gene (mutated gene), preferably said mutated gene is subject of a gain-of-function mutation.

This measure has the advantage that the composition according to the invention is adapted to eradicate the phenotypical changes of a gene mutation, especially such a mutation which may play a causative gain-of-function role in hereditary diseases.

In view of the aforesaid, in another embodiment said mutated gene is a disease-associated gene.

An example of such a gain-of-function and disease-associated mutation is a mutation in the *ELANE* gene, such as the p.A57V CN mutation.

In a further embodiment said wild-type gene is a non-essential gene.

This measure has the advantage that the targeted knockout of the gene according to the invention can, on the one hand, avoid the consequences of the expression of a potential disease-associated gene, and, on the other hand, does not result in the loss of a vital function in the affected organism.

In another embodiment of the invention the mutated gene is a mutated form of the gene encoding neutrophil elastase or elastase neutrophil expressed gene (ELANE).

This measure has the advantage that a targeted knockout of such a gene is achieved which, if mutated, is considered of being responsible for most cases of severe congenital neutropenia (CN), and cyclic neutropenia (CyN). The inventors therefore provide a genetic tool for the effective treatment of disorders of the neutrophil production, which so far cannot be cured in a satisfying manner. An example is the p.A57V CN mutation in the *ELANE* gene.

In another embodiment of the composition of the invention said first and/or second sgRNA comprises a nucleotide sequence which is selected from the group consisting of SEQ ID NOS: 1 to 252.

This measure has the advantage that sgRNA sequences are used which demonstrably guide the CRISPR endonucleases to the *ELANE* promoter, allowing the latter to be simultaneously cut in the sense and the antisense DNA strands. Such simultaneous DNA single-strand breaks result in a DNA double strand break and a functional disruption of the promotor's function. This concerted action finally results in the inhibition of the expression of the *ELANE* gene.

Another subject-matter of the invention relates to the composition according to the invention for use as a medicament, preferably for use in the treatment of an autosomal-recessive disorder, alternatively an autosomal-dominantly inherited genetic disorder, further preferably for use in the treatment of congenital neutropenia, highly preferably afore-said disorders with gain-of-function mutations.

The features, characteristics, and embodiments of the composition according to the invention apply to the composition for use as a medicament and for use in said treatment according to the invention in a corresponding manner, even if not specifically indicated.

Another subject-matter of the invention relates to a method for the targeted knockout of a gene on double-stranded DNA in a biological cell, comprising the following steps:
1) providing a biological cell comprising a gene on double-stranded DNA;
2) introducing into said biological cell a composition comprising:
   - a first CRISPR endonuclease or a nucleic acid molecule encoding the said CRISPR endonuclease;
   - a second CRISPR endonuclease or a nucleic acid molecule encoding said second CRISPR endonuclease;
      said first and said second CRISPR endonucleases are configured to create single-strand DNA breaks,
   - a first single guide RNA (sgRNA), and
   - a second sgRNA,
      said first sgRNA is configured to hybridize to the sense strand of a genetic element controlling the expression of said gene, and
      said second sgRNA is configured to hybridize to the antisense strand of said genetic element controlling the expression of said gene, and
3) incubating said cell and said composition, thereby allowing the creation of a single-strand DNA break on the sense strand of a genetic element controlling the expression of said gene and a single-strand DNA break on the antisense strand of the genetic element controlling the expression of said gene.

The features, characteristics, and embodiments of the composition according to the invention apply to this method according to the invention in a corresponding manner.

Therefore, in an embodiment of this method said composition is the composition according to the invention.

In another embodiment of this method said biological cell is a primary cell, preferably a hematopoietic stem and progenitor cell (HSPC).

By this measure therapeutic cells are generated which can be used in an autologous stem cell transplantation or *in vivo* administration, respectively. The obtained HSPCs can be a component of a hematopoietic stem cell transplant (HSCT).

In another embodiment of the method according to the invention said biological cell originates from a subject with a mutated gene, further preferably said mutated gene is subject of a gain-of-function mutation, further preferably said mutated gene is a disease-associated gene, further preferably the wild-type counterpart gene of said mutated gene is a non-essential gene, and highly preferably said mutated gene is a mutated form of the gene encoding neutrophil elastase or the elastase neutrophil expressed gene *(ELANE).*

Another subject-matter relates to a preparation comprising a biological cell prepared *in vitro* by a method comprising the following steps:
1) providing a biological cell comprising a gene on double-stranded DNA;
2) introducing into said biological cell a composition comprising:
   - a first CRISPR endonuclease or a nucleic acid molecule encoding the said CRISPR endonuclease;
   - a second CRISPR endonuclease or a nucleic acid molecule encoding said second CRISPR endonuclease;
      said first and said second CRISPR endonucleases are configured to create single-strand DNA breaks,
   - a first single guide RNA (sgRNA), and
   - a second sgRNA,
      said first sgRNA is configured to hybridize to the sense strand of a genetic element controlling the expression of said gene, and
      said second sgRNA is configured to hybridize to the antisense strand of said genetic element controlling the expression of said gene;
3) incubating said cell and said composition, thereby allowing the creation of a single-strand DNA break on the sense strand of a genetic element controlling the expression of said gene and a single-strand DNA break on the antisense strand of the genetic element controlling the expression of said gene, and
4) recovering said cell.

The features, characteristics, and embodiments of the composition according to the invention and the method for the targeted knockout according to the invention apply to the preparation according to the invention in a corresponding manner. Again, the obtained cell, such as a hematopoietic stem and progenitor cell (HSPC), can be a component of a hematopoietic stem cell transplant (HSCT).

Therefore, in an embodiment said method used for the production of the preparation is the method according to the invention.

Another subject-matter of the invention is a kit for the targeted knockout of a gene on double-stranded DNA in a biological cell, comprising the composition according to the invention and instructions for delivering the composition to said biological cell so as to knockout the gene.

A "kit" is a combination of individual elements useful for carrying out the methods of the invention, wherein the elements are optimized for use together in the methods. The kits may also contain additional reagents, chemicals, buffers, reaction vials etc. which may be useful for carrying out the methods according to the invention. Such kits unify all essential elements required to work the methods according to the invention, thus minimizing the risk of errors. Therefore, such kits also allow semi-skilled laboratory staff to perform the methods according to the invention.

The features, characteristics, advantages and embodiments specified herein apply to the kit according to the invention, even if not specifically indicated.

Another subject-matter of the invention relates to a method of treating a subject afflicted with a disease associated with a mutated gene, comprising administration of a therapeutically effective amount of the composition according to the invention.

The features, characteristics, and embodiments of the composition according to the invention apply to this method according to the invention in a corresponding manner, even if not specifically indicated.

Another subject-matter of the invention relate to a nucleic acid molecule, preferably an RNA molecule, comprising a nucleotide sequence which is selected from the group consisting of SEQ ID NOS: 1 to 252.

Still another subject-matter of the invention relates to a method of treating a subject afflicted with a disease associated with a mutated gene, preferably a dominant-autosomal disease, comprising a step of inhibiting in said subject a genetic element controlling the expression of said gene, preferably knocking-out said genetic element controlling the expression of said gene.

The features, characteristics, and embodiments of the composition according to the invention apply to this method according to the invention in a corresponding manner, even if not specifically indicated.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

### BRIEF DESCRIPTION OF THE FIGURES

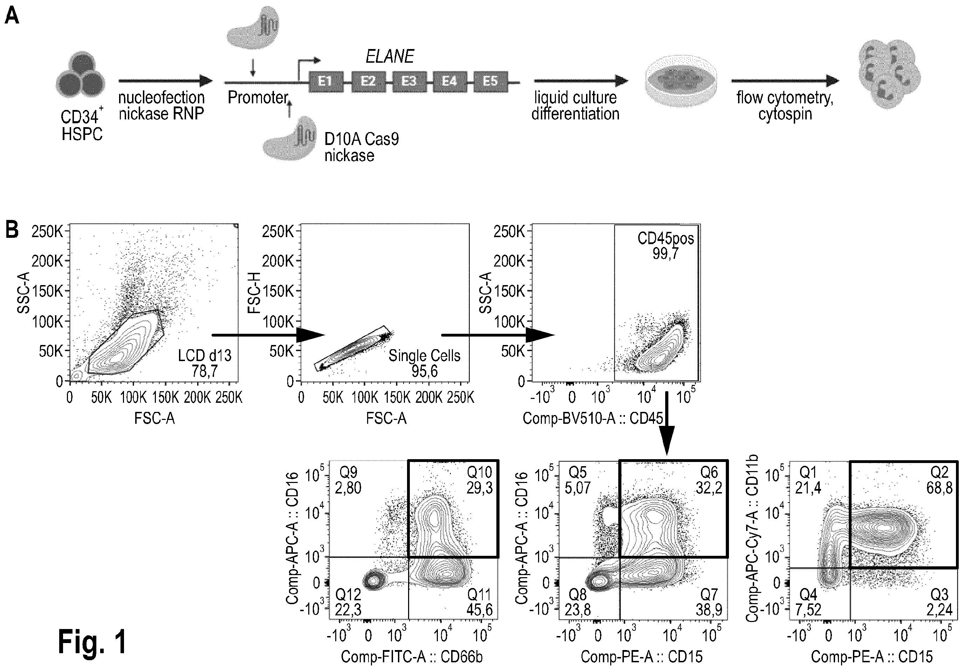

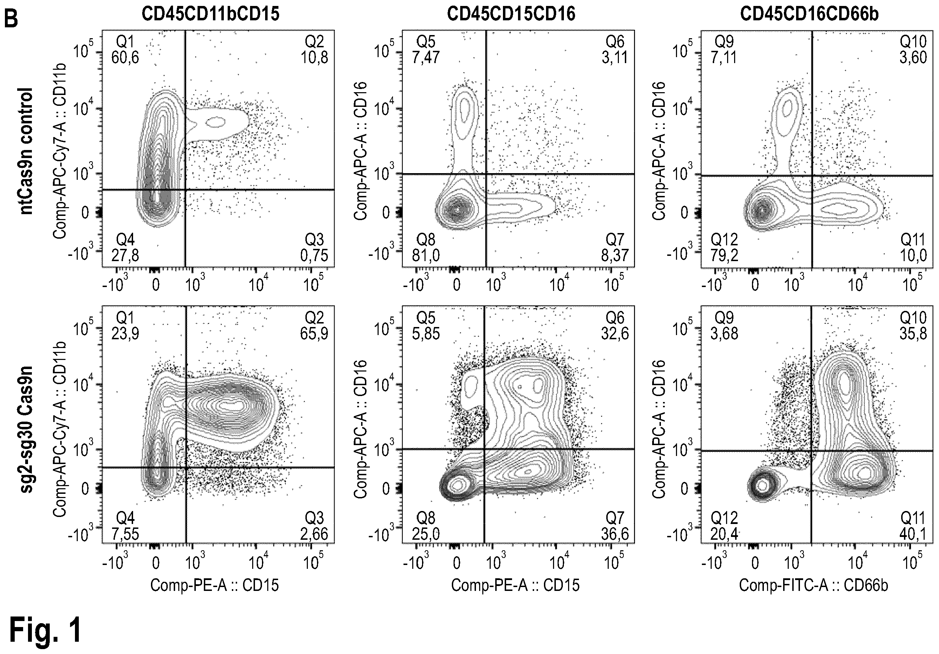

- Fig. 1:: Safe and efficient strategy to disrupt the *ELANE* gene expression by promoter knockout using CRISPR/Cas9 D10A nickase restores granulocytic differentiation of congenital neutropenia patient's HSPCs with p.A57V CN mutation. A, Scheme of the experiment. B, FACS gating strategy for cells generating during *in vitro* granulocytic differentiation C, Representative FACS density plots for granulocytic CD45⁺CD11b⁺CD15⁺, CD45⁺CD15⁺CD16⁺ and CD45⁺CD16b⁺C066b⁺ cell subsets of differentiated primary HSPCs of congenital neutropenia patient with p.A57V mutation after CRISPR/Cas9 D10A double nickases editing.

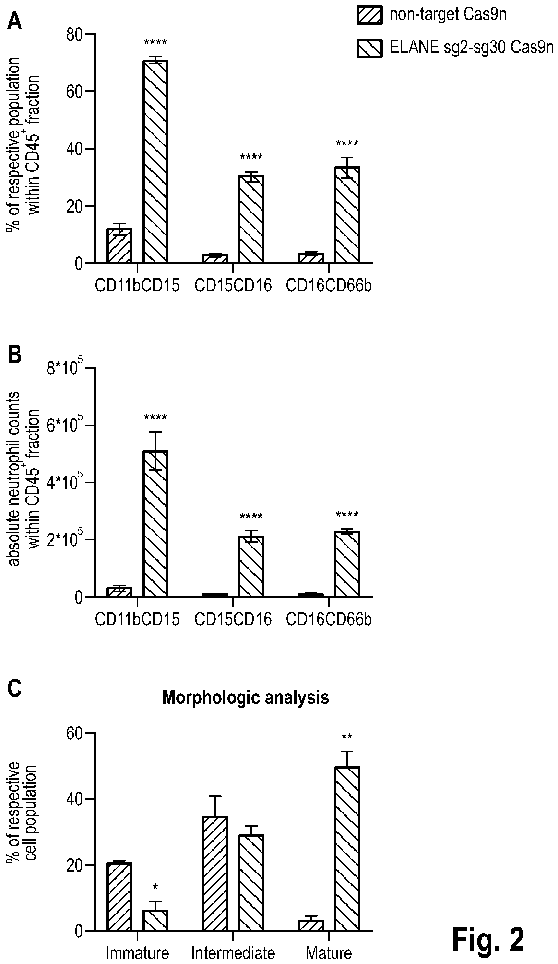

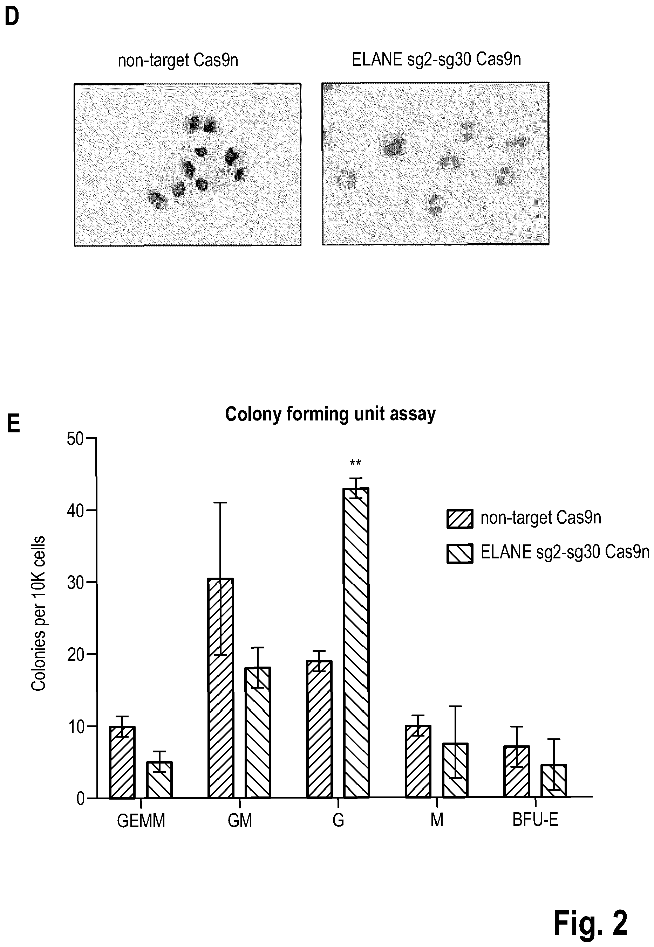

- Fig. 2:: *ELANE* promoter knockout restored granulocytic differentiation of congenital neutropenia patient's HSPCs with p.A57V CN mutation. A-D, Granulocytic differentiation capacity of *ELANE* promoter knockout HSPCs of congenital neutropenia patient's with p.A57V mutation was assessed by liquid culture differentiation after 13 days by investigating neutrophilic surface marker expression. Percentage (A), absolute cell counts (B), morphologic analysis of cytospin slides (C) and representative images of Wright-Giemsa stained cytopsins (D) are shown. E, Colony forming unit assay of the *ELANE* promoter knockout cells showed higher numbers of CFU-G compared to control cells. Data represent means ± standard deviation (SD) from triplicates. *P<0.05, **P<0.01, ****P<0.0001.

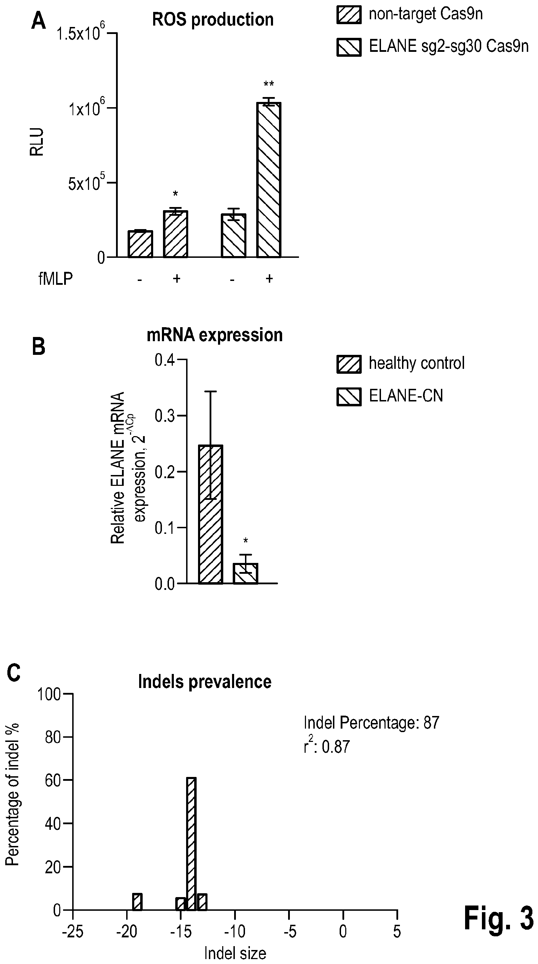

- Fig. 3:: *ELANE* promoter knockout significantly impaired the *ELANE* gene expression and improved the functionality of differentiated neutrophils. A, ROS production was significantly elevated in promoter knockout cells treated with fMLP, as compared to non-target Cas9 cells. B, ELANE mRNA expression in in vitro generated CN neutrophils was 5-fold reduced compared to unedited healthy donor neutrophils. C, Indel's prevalence was assessed by inference of CRISPR edits (ICE) at day 13 post differentiation and was 87 %.

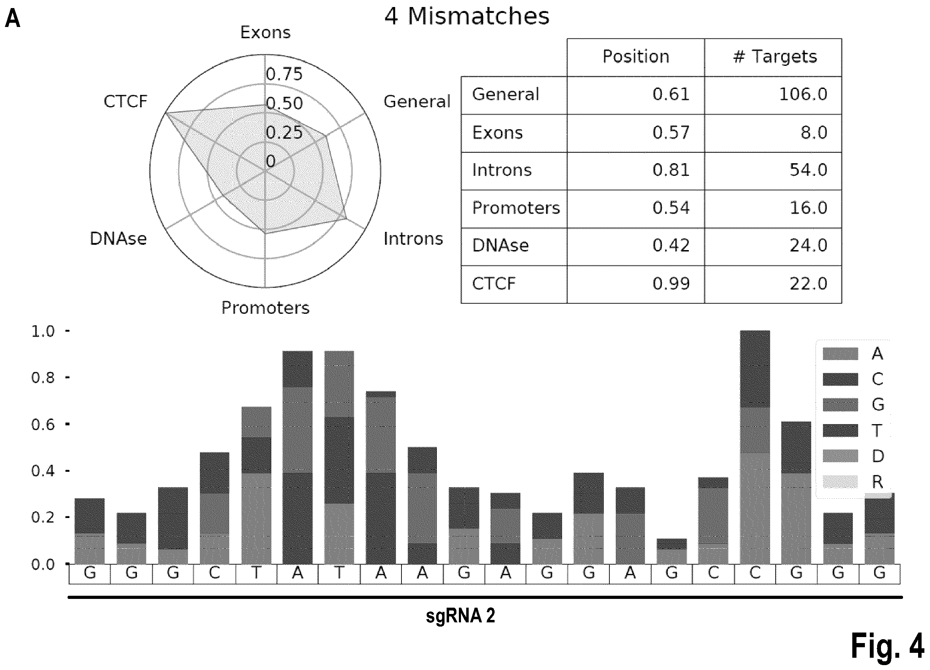

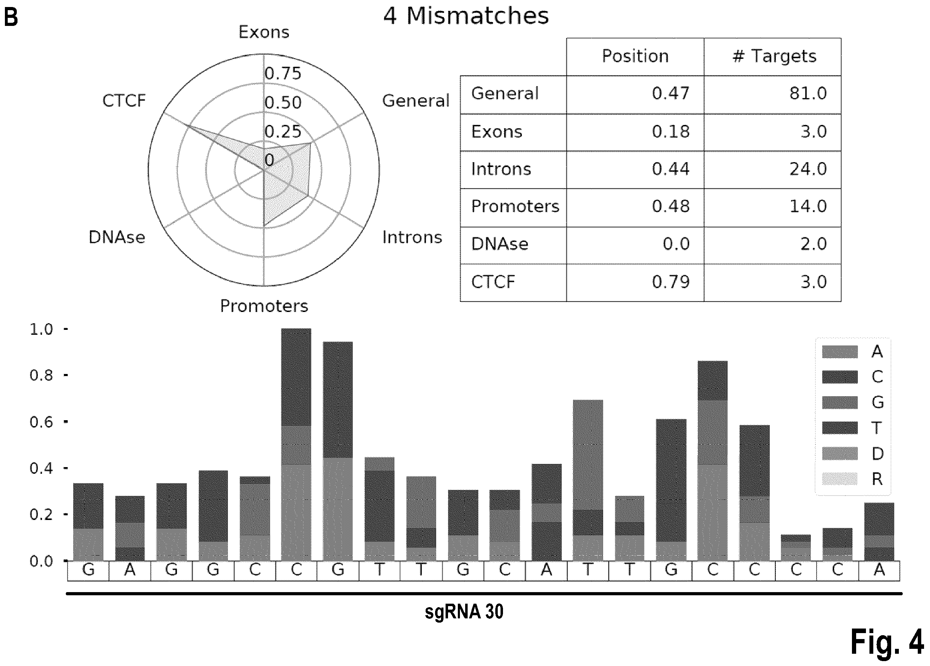

- Fig. 4:: Independent *in silico* on- and off-target profiling of sgRNA confirms the safety of the selected sgRNAs. Independent *in silico* on- and off-target profiling of each sgRNA was done using Doench 2016 algorithm using CRISPRitz tool for up to 4 mismatches using reference genome GRCh37 (hg19) for sgRNA 2 (A) and sgRNA 30 (B) shows low off-target profile for each sgRNA.

### EXAMPLES

### 1. Material and methods

### Patients

One severe congenital neutropenia patient harboring an *ELANE* mutation (*ELANE-*CN) was used in the study. Healthy individuals served as control group. Bone marrow and peripheral blood samples were collected from the patient in association with an annual follow-up recommended by the Severe Chronic Neutropenia International Registry. Study approval was obtained from the Ethical Review Board of the Medical Faculty, University of Tübingen. Informed written consent was obtained from the participant of this study.

### Cell culture

Human CD34⁺ HSPC were isolated from bone marrow mononuclear cells by magnetic bead separation using human CD34 progenitor cell isolation kit. (Miltenyi Biotech, #130-046-703). CD34⁺ cells were cultured in a density of 2 x 10⁵ cells/ml in Stemline II hematopoietic stem cell expansion medium (Sigma Aldrich, #50192) supplemented with 10 % FBS. 1 % penicillin/streptomycin, 1 % L-glutamine and a cytokine cocktail consisting of 20 ng/ml IL-3, 20 ng/ml IL-6. 20 ng/ml TPO, 50 ng/ml SCF and 50 ng/mL FLT-3L (all cytokines were purchased from R&D Systems).

### Design of the sgRNAs

Specific D10A nickase-guide-RNAs (sgRNAs) to target promoter region of *ELANE* gene (nick sites: chr19 [GGGCTATAAGAGGAGCCGGG (SEQ ID NO: 1; sgRNA 2), GAGGCCGTTGCATTGCCCCA (SEQ ID NO: 2; sgRNA 30)] were designed by using the Desktop genetics website. In total 252 sgRNAs specific for the *ELANE* promoter were developed by the inventors. They are listed in the following table along with their genomic positions according to the Genome Reference Consortium Human Build 37 (GRCh37), the specificity for the sense (1) or antisense strand (-1) and the protospacer adjacent motif ("PAM").

**Table 1: sgRNAs specific for the human ELANE promoter**

| SEQ-ID NO. | Genomic position (hg19) | Strand | Sequence | PAM |
|---|---|---|---|---|
| 1 | 852285 | 1 | GGGCTATAAGAGGAGCCGGG | CGG |
| 2 | 852243 | -1 | GAGGCCGTTGCATTGCCCCA | CGG |
| 3 | 851023 | -1 | CTCACTTTCACCCAGGCTCA | CGG |
| 4 | 851030 | -1 | CGGGGAACTCACTTTCACCC | AGG |
| 5 | 851045 | 1 | GGTGAAAGTGAGTTCCCCGT | TGG |
| 6 | 851048 | 1 | GAAAGTGAGTTCCCCGTTGG | AGG |
| 7 | 851048 | -1 | CTCGTCTGTTGCCTCCAACG | GGG |
| 8 | 851049 | -1 | CCTCGTCTGTTGCCTCCAAC | GGG |
| 9 | 851050 | -1 | TCCTCGTCTGTTGCCTCCAA | CGG |
| 10 | 851060 | 1 | CCCGTTGGAGGCAACAGACG | AGG |
| 11 | 851065 | 1 | TGGAGGCAACAGACGAGGAG | AGG |
| 12 | 851069 | 1 | GGCAACAGACGAGGAGAGGA | TGG |
| 13 | 851073 | 1 | ACAGACGAGGAGAGGATGGA | AGG |
| 14 | 851078 | 1 | CGAGGAGAGGATGGAAGGCC | TGG |
| 15 | 851085 | -1 | AGGGCTCATTCTTGGGGGCC | AGG |
| 16 | 851090 | -1 | ACCTCAGGGCTCATTCTTGG | GGG |
| 17 | 851091 | -1 | AACCTCAGGGCTCATTCTTG | GGG |
| 18 | 851092 | -1 | GAACCTCAGGGCTCATTCTT | GGG |
| 19 | 851093 | -1 | TGAACCTCAGGGCTCATTCT | TGG |
| 20 | 851100 | 1 | GCCCCCAAGAATGAGCCCTG | AGG |
| 21 | 851104 | -1 | CAGCCGCTCCCTGAACCTCA | GGG |
| 22 | 851105 | -1 | CCAGCCGCTCCCTGAACCTC | AGG |
| 23 | 851106 | 1 | AAGAATGAGCCCTGAGGTTC | AGG |
| 24 | 851107 | 1 | AGAATGAGCCCTGAGGTTCA | GGG |
| 25 | 851112 | 1 | GAGCCCTGAGGTTCAGGGAG | CGG |
| 26 | 851116 | 1 | CCTGAGGTTCAGGGAGCGGC | TGG |
| 27 | 851126 | 1 | AGGGAGCGGCTGGAGTGAGC | CGG |
| 28 | 851134 | -1 | GCTGGACGGAGATCTGGGGC | CGG |
| 29 | 851138 | -1 | CGCAGCTGGACGGAGATCTG | GGG |
| 30 | 851139 | -1 | CCGCAGCTGGACGGAGATCT | GGG |
| 31 | 851140 | -1 | CCCGCAGCTGGACGGAGATC | TGG |
| 32 | 851148 | -1 | CTCTGGGACCCGCAGCTGGA | CGG |
| 33 | 851150 | 1 | CCCAGATCTCCGTCCAGCTG | CGG |
| 34 | 851151 | 1 | CCAGATCTCCGTCCAGCTGC | GGG |
| 35 | 851152 | -1 | AGGCCTCTGGGACCCGCAGC | TGG |
| 36 | 851160 | 1 | CGTCCAGCTGCGGGTCCCAG | AGG |
| 37 | 851164 | -1 | CGAGTGTAACCCAGGCCTCT | GGG |
| 38 | 851165 | 1 | AGCTGCGGGTCCCAGAGGCC | TGG |
| 39 | 851165 | -1 | GCGAGTGTAACCCAGGCCTC | TGG |
| 40 | 851166 | 1 | GCTGCGGGTCCCAGAGGCCT | GGG |
| 41 | 851172 | -1 | AGGAGCTGCGAGTGTAACCC | AGG |
| 42 | 851185 | 1 | TGGGTTACACTCGCAGCTCC | TGG |
| 43 | 851186 | 1 | GGGTTACACTCGCAGCTCCT | GGG |
| 44 | 851187 | 1 | GGTTACACTCGCAGCTCCTG | GGG |
| 45 | 851188 | 1 | GTTACACTCGCAGCTCCTGG | GGG |
| 46 | 851191 | 1 | ACACTCGCAGCTCCTGGGGG | AGG |
| 47 | 851192 | -1 | GCACGTCAAGGGCCTCCCCC | AGG |
| 48 | 851203 | -1 | TGGGAACTGAGGCACGTCAA | GGG |
| 49 | 851204 | -1 | TTGGGAACTGAGGCACGTCA | AGG |
| 50 | 851214 | -1 | GGGTTCCTGTTTGGGAACTG | AGG |
| 51 | 851220 | 1 | ACGTGCCTCAGTTCCCAAAC | AGG |
| 52 | 851222 | -1 | CCTTCCCAGGGTTCCTGTTT | GGG |
| 53 | 851223 | -1 | TCCTTCCCAGGGTTCCTGTT | TGG |
| 54 | 851228 | 1 | CAGTTCCCAAACAGGAACCC | TGG |
| 55 | 851229 | 1 | AGTTCCCAAACAGGAACCCT | GGG |
| 56 | 851233 | 1 | CCCAAACAGGAACCCTGGGA | AGG |
| 57 | 851234 | -1 | CACTTCTCTGGTCCTTCCCA | GGG |
| 58 | 851235 | -1 | GCACTTCTCTGGTCCTTCCC | AGG |
| 59 | 851246 | -1 | CTGCGCAATAGGCACTTCTC | TGG |
| 60 | 851257 | -1 | TCGGGCACTCACTGCGCAAT | AGG |
| 61 | 851275 | -1 | CGGCCACATGCAGCTGTGTC | GGG |
| 62 | 851276 | -1 | CCGGCCACATGCAGCTGTGT | CGG |
| 63 | 851283 | 1 | GTGCCCGACACAGCTGCATG | TGG |
| 64 | 851287 | 1 | CCGACACAGCTGCATGTGGC | CGG |
| 65 | 851295 | -1 | TACCCAGGGCCCTGTGATAC | CGG |
| 66 | 851296 | 1 | CTGCATGTGGCCGGTATCAC | AGG |
| 67 | 851297 | 1 | TGCATGTGGCCGGTATCACA | GGG |
| 68 | 851303 | 1 | TGGCCGGTATCACAGGGCCC | TGG |
| 69 | 851304 | 1 | GGCCGGTATCACAGGGCCCT | GGG |
| 70 | 851309 | -1 | CGCCTGCCTCAGTTTACCCA | GGG |
| 71 | 851310 | -1 | TCGCCTGCCTCAGTTTACCC | AGG |
| 72 | 851314 | 1 | ACAGGGCCCTGGGTAAACTG | AGG |
| 73 | 851318 | 1 | GGCCCTGGGTAAACTGAGGC | AGG |
| 74 | 851336 | 1 | GCAGGCGACACAGCTGCATG | TGG |
| 75 | 851340 | 1 | GCGACACAGCTGCATGTGGC | CGG |
| 76 | 851348 | -1 | TACCCAGGGCCCTGTGATAC | CGG |
| 77 | 851349 | 1 | CTGCATGTGGCCGGTATCAC | AGG |
| 78 | 851350 | 1 | TGCATGTGGCCGGTATCACA | GGG |
| 79 | 851356 | 1 | TGGCCGGTATCACAGGGCCC | TGG |
| 80 | 851357 | 1 | GGCCGGTATCACAGGGCCCT | GGG |
| 81 | 851362 | -1 | CGCCTGCCTCAGTTTACCCA | GGG |
| 82 | 851363 | -1 | TCGCCTGCCTCAGTTTACCC | AGG |
| 83 | 851367 | 1 | ACAGGGCCCTGGGTAAACTG | AGG |
| 84 | 851371 | 1 | GGCCCTGGGTAAACTGAGGC | AGG |
| 85 | 851389 | 1 | GCAGGCGACACAGCTGCATG | TGG |
| 86 | 851393 | 1 | GCGACACAGCTGCATGTGGC | CGG |
| 87 | 851401 | -1 | TACCCAGGGCCCTGTGATAC | CGG |
| 88 | 851402 | 1 | CTGCATGTGGCCGGTATCAC | AGG |
| 89 | 851403 | 1 | TGCATGTGGCCGGTATCACA | GGG |
| 90 | 851409 | 1 | TGGCCGGTATCACAGGGCCC | TGG |
| 91 | 851410 | 1 | GGCCGGTATCACAGGGCCCT | GGG |
| 92 | 851415 | -1 | CGCCTGCCTCAGTTTACCCA | GGG |
| 93 | 851416 | -1 | TCGCCTGCCTCAGTTTACCC | AGG |
| 94 | 851420 | 1 | ACAGGGCCCTGGGTAAACTG | AGG |
| 95 | 851424 | 1 | GGCCCTGGGTAAACTGAGGC | AGG |
| 96 | 851442 | 1 | GCAGGCGACACAGCTGCATG | TGG |
| 97 | 851454 | 1 | GCTGCATGTGGCCGTATCAC | AGG |
| 98 | 851454 | -1 | TTACCCAGGGCCCTGTGATA | CGG |
| 99 | 851455 | 1 | CTGCATGTGGCCGTATCACA | GGG |
| 100 | 851461 | 1 | GTGGCCGTATCACAGGGCCC | TGG |
| 101 | 851462 | 1 | TGGCCGTATCACAGGGCCCT | GGG |
| 102 | 851467 | -1 | CACCTGCCTCAGTTTACCCA | GGG |
| 103 | 851468 | -1 | TCACCTGCCTCAGTTTACCC | AGG |
| 104 | 851472 | 1 | ACAGGGCCCTGGGTAAACTG | AGG |
| 105 | 851476 | 1 | GGCCCTGGGTAAACTGAGGC | AGG |
| 106 | 851494 | 1 | GCAGGTGACACAGCTGCATG | TGG |
| 107 | 851498 | 1 | GTGACACAGCTGCATGTGGC | CGG |
| 108 | 851506 | 1 | GCTGCATGTGGCCGGTATCA | CGG |
| 109 | 851506 | -1 | TATCCAGGGCCCCGTGATAC | CGG |
| 110 | 851507 | 1 | CTGCATGTGGCCGGTATCAC | GGG |
| 111 | 851508 | 1 | TGCATGTGGCCGGTATCACG | GGG |
| 112 | 851514 | 1 | TGGCCGGTATCACGGGGCCC | TGG |
| 113 | 851520 | -1 | CGCCTGCCTCTGTTTATCCA | GGG |
| 114 | 851521 | -1 | TCGCCTGCCTCTGTTTATCC | AGG |
| 115 | 851525 | 1 | ACGGGGCCCTGGATAAACAG | AGG |
| 116 | 851529 | 1 | GGCCCTGGATAAACAGAGGC | AGG |
| 117 | 851547 | 1 | GCAGGCGACACAGCTGCATG | TGG |
| 118 | 851551 | 1 | GCGACACAGCTGCATGTGGC | CGG |
| 119 | 851559 | 1 | GCTGCATGTGGCCGGTATCA | CGG |
| 120 | 851559 | -1 | TACCCAGGGCCCCGTGATAC | CGG |
| 121 | 851560 | 1 | CTGCATGTGGCCGGTATCAC | GGG |
| 122 | 851561 | 1 | TGCATGTGGCCGGTATCACG | GGG |
| 123 | 851567 | 1 | TGGCCGGTATCACGGGGCCC | TGG |
| 124 | 851568 | 1 | GGCCGGTATCACGGGGCCCT | GGG |
| 125 | 851573 | -1 | CGCCTGCCTCAGTTTACCCA | GGG |
| 126 | 851574 | -1 | TCGCCTGCCTCAGTTTACCC | AGG |
| 127 | 851578 | 1 | ACGGGGCCCTGGGTAAACTG | AGG |
| 128 | 851582 | 1 | GGCCCTGGGTAAACTGAGGC | AGG |
| 129 | 851587 | 1 | TGGGTAAACTGAGGCAGGCG | AGG |
| 130 | 851599 | -1 | CCTGAGGGACTTGATGGGGG | TGG |
| 131 | 851602 | -1 | AGACCTGAGGGACTTGATGG | GGG |
| 132 | 851603 | -1 | TAGACCTGAGGGACTTGATG | GGG |
| 133 | 851604 | -1 | CTAGACCTGAGGGACTTGAT | GGG |
| 134 | 851605 | -1 | CCTAGACCTGAGGGACTTGA | TGG |
| 135 | 851610 | 1 | CCACCCCCATCAAGTCCCTC | AGG |
| 136 | 851614 | -1 | CCTGCCAAACCTAGACCTGA | GGG |
| 137 | 851615 | -1 | ACCTGCCAAACCTAGACCTG | AGG |
| 138 | 851616 | 1 | CCATCAAGTCCCTCAGGTCT | AGG |
| 139 | 851621 | 1 | AAGTCCCTCAGGTCTAGGTT | TGG |
| 140 | 851625 | 1 | CCCTCAGGTCTAGGTTTGGC | AGG |
| 141 | 851630 | 1 | AGGTCTAGGTTTGGCAGGTT | TGG |
| 142 | 851651 | 1 | GGCAAAAACACAGCAACGCT | CGG |
| 143 | 851668 | 1 | GCTCGGTTAAATCTGAATTT | CGG |
| 144 | 851669 | 1 | CTCGGTTAAATCTGAATTTC | GGG |
| 145 | 851683 | 1 | AATTTCGGGTAAGTATATCC | TGG |
| 146 | 851684 | 1 | ATTTCGGGTAAGTATATCCT | GGG |
| 147 | 851690 | -1 | GTCTCTTCCAAATGAGGCCC | AGG |
| 148 | 851694 | 1 | AGTATATCCTGGGCCTCATT | TGG |
| 149 | 851696 | -1 | ATCTAAGTCTCTTCCAAATG | AGG |
| 150 | 851735 | 1 | AAAAAACGTCGAGACCAGCC | CGG |
| 151 | 851738 | -1 | TTTCACCGTGTTGGCCGGGC | TGG |
| 152 | 851742 | -1 | GGGGTTTCACCGTGTTGGCC | GGG |
| 153 | 851743 | -1 | CGGGGTTTCACCGTGTTGGC | CGG |
| 154 | 851744 | 1 | CGAGACCAGCCCGGCCAACA | CGG |
| 155 | 851747 | -1 | GAGACGGGGTTTCACCGTGT | TGG |
| 156 | 851761 | -1 | TTGTATTTTTAGTAGAGACG | GGG |
| 157 | 851762 | -1 | TTTGTATTTTTAGTAGAGAC | GGG |
| 158 | 851763 | -1 | TTTTGTATTTTTAGTAGAGA | CGG |
| 159 | 851785 | 1 | AAAAATACAAAAAATTAGCC | AGG |
| 160 | 851792 | -1 | CAGGCGTGAGCCACTGCGCC | TGG |
| 161 | 851793 | 1 | AAAAAATTAGCCAGGCGCAG | TGG |
| 162 | 851811 | -1 | TCCCAGAGTGCTGGGATCAC | AGG |
| 163 | 851819 | -1 | CCTCAGCCTCCCAGAGTGCT | GGG |
| 164 | 851820 | 1 | CGCCTGTGATCCCAGCACTC | TGG |
| 165 | 851820 | -1 | GCCTCAGCCTCCCAGAGTGC | TGG |
| 166 | 851821 | 1 | GCCTGTGATCCCAGCACTCT | GGG |
| 167 | 851824 | 1 | TGTGATCCCAGCACTCTGGG | AGG |
| 168 | 851830 | 1 | CCCAGCACTCTGGGAGGCTG | AGG |
| 169 | 851834 | 1 | GCACTCTGGGAGGCTGAGGC | AGG |
| 170 | 851837 | 1 | CTCTGGGAGGCTGAGGCAGG | CGG |
| 171 | 851848 | 1 | TGAGGCAGGCGGATCACCCG | AGG |
| 172 | 851853 | -1 | GGTCTTGAACATCTGACCTC | GGG |
| 173 | 851854 | -1 | TGGTCTTGAACATCTGACCT | CGG |
| 174 | 851871 | 1 | TCAGATGTTCAAGACCAGCC | TGG |
| 175 | 851874 | -1 | TTTCGCCCTGTCGGCCAGGC | TGG |
| 176 | 851878 | -1 | AGTGTTTCGCCCTGTCGGCC | AGG |
| 177 | 851879 | 1 | TCAAGACCAGCCTGGCCGAC | AGG |
| 178 | 851880 | 1 | CAAGACCAGCCTGGCCGACA | GGG |
| 179 | 851883 | -1 | GAGACAGTGTTTCGCCCTGT | CGG |
| 180 | 851919 | 1 | CTACAAATACAAAAATTAGC | CGG |
| 181 | 851920 | 1 | TACAAATACAAAAATTAGCC | GGG |
| 182 | 851925 | 1 | ATACAAAAATTAGCCGGGAG | TGG |
| 183 | 851927 | -1 | CAGGCACCTGCCACCACTCC | CGG |
| 184 | 851928 | 1 | CAAAAATTAGCCGGGAGTGG | TGG |
| 185 | 851932 | 1 | AATTAGCCGGGAGTGGTGGC | AGG |
| 186 | 851946 | -1 | TCCTGAATAGCTGAGATTAC | AGG |
| 187 | 851956 | 1 | GCCTGTAATCTCAGCTATTC | AGG |
| 188 | 851959 | 1 | TGTAATCTCAGCTATTCAGG | AGG |
| 189 | 851965 | 1 | CTCAGCTATTCAGGAGGCTG | AGG |
| 190 | 851969 | 1 | GCTATTCAGGAGGCTGAGGC | AGG |
| 191 | 851987 | 1 | GCAGGAGAATCACTTGAACC | TGG |
| 192 | 851988 | 1 | CAGGAGAATCACTTGAACCT | GGG |
| 193 | 851991 | 1 | GAGAATCACTTGAACCTGGG | AGG |
| 194 | 851994 | 1 | AATCACTTGAACCTGGGAGG | CGG |
| 195 | 851994 | -1 | CACGGCAACCTCCGCCTCCC | AGG |
| 196 | 851997 | 1 | CACTTGAACCTGGGAGGCGG | AGG |
| 197 | 852011 | 1 | AGGCGGAGGTTGCCGTGAGC | CGG |
| 198 | 852012 | 1 | GGCGGAGGTTGCCGTGAGCC | GGG |
| 199 | 852012 | -1 | GGTGGCGTGATCCCGGCTCA | CGG |
| 200 | 852019 | -1 | GGAGTGCGGTGGCGTGATCC | CGG |
| 201 | 852030 | -1 | TCGCCCAGGCTGGAGTGCGG | TGG |
| 202 | 852033 | -1 | CTATCGCCCAGGCTGGAGTG | CGG |
| 203 | 852037 | 1 | CACGCCACCGCACTCCAGCC | TGG |
| 204 | 852038 | 1 | ACGCCACCGCACTCCAGCCT | GGG |
| 205 | 852040 | -1 | TCTTGCTCTATCGCCCAGGC | TGG |
| 206 | 852044 | -1 | AGAGTCTTGCTCTATCGCCC | AGG |
| 207 | 852071 | -1 | GGTTTTTTAATTTATTTTTT | TGG |
| 208 | 852092 | -1 | AAATGTCAGATAATCAATGT | GGG |
| 209 | 852093 | -1 | CAAATGTCAGATAATCAATG | TGG |
| 210 | 852131 | -1 | GGGGCCTCCAGACAAAATTC | AGG |
| 211 | 852135 | 1 | TGTGCATCCTGAATTTTGTC | TGG |
| 212 | 852138 | 1 | GCATCCTGAATTTTGTCTGG | AGG |
| 213 | 852150 | -1 | ACGCTGGATTGGCTCGGGTG | GGG |
| 214 | 852151 | -1 | GACGCTGGATTGGCTCGGGT | GGG |
| 215 | 852152 | -1 | AGACGCTGGATTGGCTCGGG | TGG |
| 216 | 852155 | -1 | ACAAGACGCTGGATTGGCTC | GGG |
| 217 | 852156 | -1 | GACAAGACGCTGGATTGGCT | CGG |
| 218 | 852161 | -1 | AGGGGGACAAGACGCTGGAT | TGG |
| 219 | 852166 | -1 | GGAGAAGGGGGACAAGACGC | TGG |
| 220 | 852178 | -1 | TGATGAAAAGGGGGAGAAGG | GGG |
| 221 | 852179 | -1 | TTGATGAAAAGGGGGAGAAG | GGG |
| 222 | 852180 | -1 | GTTGATGAAAAGGGGGAGAA | GGG |
| 223 | 852181 | -1 | CGTTGATGAAAAGGGGGAGA | AGG |
| 224 | 852187 | -1 | ACAGGGCGTTGATGAAAAGG | GGG |
| 225 | 852188 | -1 | CACAGGGCGTTGATGAAAAG | GGG |
| 226 | 852189 | -1 | GCACAGGGCGTTGATGAAAA | GGG |
| 227 | 852190 | -1 | GGCACAGGGCGTTGATGAAA | AGG |
| 228 | 852204 | 1 | TTTCATCAACGCCCTGTGCC | AGG |
| 229 | 852204 | -1 | ACTTCCTCTCCCCTGGCACA | GGG |
| 230 | 852205 | 1 | TTCATCAACGCCCTGTGCCA | GGG |
| 231 | 852205 | -1 | CACTTCCTCTCCCCTGGCAC | AGG |
| 232 | 852206 | 1 | TCATCAACGCCCTGTGCCAG | GGG |
| 233 | 852211 | 1 | AACGCCCTGTGCCAGGGGAG | AGG |
| 234 | 852211 | -1 | GCCCTCCACTTCCTCTCCCC | TGG |
| 235 | 852217 | 1 | CTGTGCCAGGGGAGAGGAAG | TGG |
| 236 | 852220 | 1 | TGCCAGGGGAGAGGAAGTGG | AGG |
| 237 | 852221 | 1 | GCCAGGGGAGAGGAAGTGGA | GGG |
| 238 | 852227 | 1 | GGAGAGGAAGTGGAGGGCGC | TGG |
| 239 | 852231 | 1 | AGGAAGTGGAGGGCGCTGGC | CGG |
| 240 | 852237 | 1 | TGGAGGGCGCTGGCCGGCCG | TGG |
| 241 | 852238 | 1 | GGAGGGCGCTGGCCGGCCGT | GGG |
| 242 | 852239 | 1 | GAGGGCGCTGGCCGGCCGTG | GGG |
| 243 | 852239 | -1 | CCGTTGCATTGCCCCACGGC | CGG |
| 244 | 852250 | 1 | CCGGCCGTGGGGCAATGCAA | CGG |
| 245 | 852262 | -1 | TCTTATAGCCCTGTGCTGGG | AGG |
| 246 | 852264 | 1 | ATGCAACGGCCTCCCAGCAC | AGG |
| 247 | 852265 | 1 | TGCAACGGCCTCCCAGCACA | GGG |
| 248 | 852265 | -1 | TCCTCTTATAGCCCTGTGCT | GGG |
| 249 | 852266 | -1 | CTCCTCTTATAGCCCTGTGC | TGG |
| 250 | 852275 | 1 | TCCCAGCACAGGGCTATAAG | AGG |
| 251 | 852281 | 1 | CACAGGGCTATAAGAGGAGC | CGG |
| 252 | 852282 | 1 | ACAGGGCTATAAGAGGAGCC | GGG |

### CRISPR/Cas9 D10A nickase-gRNA RNP mediated ELANE promoter KO in HSPC

Nucleofection was carried out using the Amaxa nucleofection system (P3 primary kit, #V4XP-3024) according to the manufacturer's instructions. For 1 x 10⁶ human CD34⁺ HSPC, 300 pmol sgRNA and 300 pmol Hifi Cas9 protein (Integrated DNA Technologies) was used for nucleofection.

### Assessing genome editing efficiency

Gene editing efficiency assessed either by Sanger sequencing of the promoter region qRT-PCR.

### RNA isolation, cDNA synthesis and qRT-PCR

RNA was isolated using RNeasy Mini Kit (Qiagen) and cDNA was prepared using Omniscript RT kit (Qiagen). qPCR was performed using SYBR Green qPCR master mix (Roche) and Light Cycler 480 (Roche). *ELANE* [primers: Fwd: GTGTCWTCCTCGCCTGTGTC (SEQ ID NO: 253), Rev: CCCACAATCTCCGAGGCCAG (SEQ ID NO: 254)] mRNA expression was normalized to GAPDH [primers: Fwd: CTGGGCTACACTGAGCACC (SEQ ID NO: 255), Rev: AAGTGGTCGTTGAGGGCAATG (SEQ ID NO: 256)] and for ACTB [primers: Fwd: CATGTACGTTGCTATCCAGGC (SEQ ID NO: 257), Rev: CTCCTTAATGTCACGCACGAT (SEQ ID NO: 258)] mRNA expression levels.

### Liquid culture differentiation of CD34⁺ cells

CD34⁺ cells were seeded in a density of 2 x 10⁵ cells/ml. Cells were incubated for 7 days in RPMI 1640 GlutaMAX supplemented with 10 % FBS. 1 % penicillin/streptomycin, 5 ng/ml SCF, 5 ng/ml IL-3, 5 ng/ml GM-CSF and 10 ng/ml G-CSF. Medium was exchanged every second day. On day 7, cells were plated in RPMI 1640 GlutaMAX supplemented with 10 % FBS, 1 % penicillin/streptomycin and 10 ng/ml G-CSF. On day 14, cells were analyzed by FACS using following mouse anti-human antibodies: CD34 (BB, #343811). CD33 (BioLegend, #303416), CD45 (BioLegend, #304036), CD11b (BD, #557754), CD15 (BE, #555402), CD16 (BD, #561248), CD66b (Biolegend, #305104). Morphology of the cells was investigated on cytospin slides by Wright-Giemsa staining.

### 2. Results

To perform *ELANE* transcriptional repression using CRISPR/Cas9 D10A nickase, HSPCs of an *ELANE-*CN patient with a p.A57V mutation were nucleofected with CRISPR/Cas9 D10A RNP and two days later cells were processed for liquid culture differentiation towards neutrophils (Figure 1A). The same approach was used with healthy volunteers (data not shown).

Using the double nick strategy in *ELANE-*CN HSPCs, the inventors observed markedly elevated neutrophil differentiation, as assessed by the percentage of CD45⁺CD11b⁺CD15⁺, CD45⁺CD15⁺CD16⁺ and CD45⁺CD16⁺CD66b⁺ cells (Figure 1B, C and 2A, B) compared to *ELANE-*CN control cells nucleofected with non-targeting CRISPR/Cas9 D10A RNP. No effects were observed with healthy volunteers (data not shown).

Again, comparing morphological analysis of *ELANE-*CN with promoter KO to control cells (see above) the strongly improved number of mature granulocytes was confirmed (Figure 2C, D).

Similar results were achieved by colony forming unit assay (CFU) showing significantly more granulocytic colony-forming units (CFU-G colonies) in the *ELANE* promoter KO cells compared to non-targeting CRISPR/Cas9 D10A RNP control (Figure 2E). Again, effects were observed with healthy volunteers (data not shown).

The inventors further tested the functionality of *in vitro* generated *ELANE* promoter KO neutrophils by testing the reactive oxygen species (ROS) production in response to fMLP. Indeed, *ELANE* promoter KO cells produced significantly more ROS) in response to fMLP, as compared to control cells with non-targeting CRISPR/Cas9 D10A RNP (Figure 3A).

*ELANE* mRNA expression was reduced by around 5-fold in neutrophils from *ELANE* promoter KO cells compared to neutrophils from healthy donors (Figure 3B). Indel's prevalence at targeted site was 87% as assessed by inference of CRISPR edits (ICE) from Sanger Sequencing Trace Data (Figure 3C), indicating high gene-editing efficiency.

No effects were observed with healthy volunteers (data not shown).

The inventors also performed an independent *in silico* on- and off-target profiling of each sgRNA for up to 4 mismatches using Doench 2016 algorithm of CRISPRitz tool (Figure 4A, B). The inventors found low off-target effects for each single sgRNAs, indicating very low off-target effects of the combination of both sgRNAs (Figure 4A, B).

### 3. Conclusion

Taken together, the inventors established a safe approach for reducing a gene's expression by CRISPR/Cas9 double nick strategy without affecting the gene's coding sequence. This approach was exemplified as feasible using the *ELANE* gene involved in the development of congenital neutropenia. As demonstrated by examples the approach according to the invention can rescue granulopoiesis in *ELANE-*CN patients. In healthy individuals the knock-out of the *ELANE* promoter has no effect on granulopoiesis. Therefore, the invention offers a long-term treatment option replacing the daily G-CSF therapy and decreasing the risk of leukemia development.

Neutrophil elastase (NE) is a proteolytic enzyme of the neutrophil serine protease (NSP) family, including also cathepsin G (CG), proteinase 3 (PR3) and azurocidin (AZU1). NSPs are stored in cytoplasmic granules, can be secreted into the extra- and peri-cellular space upon cellular activation and considered to be crucially involved in bacterial defense. Elane^{-/-} mice have normal neutrophil counts, but there are conflicting results regarding the effect of NE-deficiency on neutrophil extravasation to sites of inflammation, phagocytosis, and neutrophil extracellular traps in mice. NE may or may not be essential for these processes. Papillon-Lefevre Syndrome (PLS) is a human disorder known to cause NE deficiency. This rare autosomal recessive disease is due to loss-of-function mutations in the DPPI gene locus with the loss of the lysosomal cysteine protease cathepsin C/dipeptidyl peptidase I (DPPI). The activation NSP, including NE, depends on the N-terminal processing activity of DPPI. Therefore, PLS patients exhibit a severe reduction in the activity and stability of all three NSP. Intriguingly, patients with PLS have no defects in their ability to kill bacteria e.g. *Staphylococcus aureus* or *Escherichia coli,* suggesting that redundancies in the neutrophil's bactericidal mechanisms negate the necessity for serine proteases for killing common bacteria. Based on these observations, at this juncture, the inventors believe that CRISPR/Cas9 based knockout of *ELANE* in HSPC of CN patients may restore defective granulopoiesis in CN patients without seriously impairing neutrophil functions.

## Claims

1. A composition for the targeted knockout of a gene on double-stranded DNA in a biological cell, comprising:
- a first CRISPR endonuclease or a nucleic acid molecule encoding the said CRISPR endonuclease;
- a second CRISPR endonuclease or a nucleic acid molecule encoding said second CRISPR endonuclease;
said first and said second CRISPR endonucleases are configured to create single-strand DNA breaks,
- a first single guide RNA (sgRNA), and
- a second sgRNA,
said first sgRNA is configured to hybridize to the sense strand of a genetic element controlling the expression of said gene, and
said second sgRNA is configured to hybridize to the antisense strand of said genetic element controlling the expression of said gene.

2. The composition of claim 2, wherein said genetic element controlling the expression of said gene is a promoter.

3. The composition of claim 1 or 2, wherein said first and/or second CRISPR endonuclease is a variant of CRISPR associated protein 9 (Cas9), preferably said variant of Cas9 comprises mutation(s) in the nuclease domain(s) RuvC and/or HNH, said mutation(s) conferring DNA nickase activity, further preferably said variant of Cas 9 is Cas9 D10A nickase and/or Cas9 H840A nickase.

4. The composition of any of claims 1 to 3, wherein said gene is a mutated form of a wild type gene (mutated gene), preferably said mutated gene is subject of a gain-of-function mutation, further preferably said mutated gene is a disease-associated gene, further preferably said wild-type gene is a non-essential gene, and highly preferably said mutated gene is a mutated form of the gene encoding neutrophil elastase (*ELANE*)*.*

5. The composition of any of claims 1 to 10, wherein said first and/or second sgRNA comprises the nucleotide sequence which is selected from the group consisting of SEQ ID NOS: 1 to 252.

6. The composition of any of claims 1 to 11 for use as a medicament, preferably for use in the treatment of an autosomal-recessive disorder, alternatively an autosomal-dominantly inherited genetic disorder, further preferably for use in the treatment of congenital neutropenia, highly preferably afore-said disorders with gain-of-function mutations.

7. A method for the targeted knockout of a gene on double-stranded DNA in a biological cell, comprising the following steps:
1) providing a biological cell comprising a gene on double-stranded DNA;
2) introducing into said biological cell a composition comprising:
- a first CRISPR endonuclease or a nucleic acid molecule encoding the said CRISPR endonuclease;
- a second CRISPR endonuclease or a nucleic acid molecule encoding said second CRISPR endonuclease;
said first and said second CRISPR endonucleases are configured to create single-strand DNA breaks,
- a first single guide RNA (sgRNA), and
- a second sgRNA,
said first sgRNA is configured to hybridize to the sense strand of a genetic element controlling the expression of said gene, and
said second sgRNA is configured to hybridize to the antisense strand of said genetic element controlling the expression of said gene, and
3) incubating said cell and said composition, thereby allowing the creation of a single-strand DNA break on the sense strand of a genetic element controlling the expression of said gene and a single-strand DNA break on the antisense strand of the genetic element controlling the expression of said gene.

8. The method of claim 7, wherein said composition is the composition of any of claims 1 to 5.

9. The method of claim 7 or 8, wherein said biological cell is a primary cell, preferably a hematopoietic stem and progenitor cell (HSPC), further preferably said biological cell originates from a subject with a mutated gene, further preferably said mutated gene is subject of a gain-of-function mutation, further preferably said mutated gene is a disease-associated gene, further preferably the wild-type counterpart gene of said mutated gene is a non-essential gene, and highly preferably said mutated gene is a mutated form of the gene encoding neutrophil elastase (*ELANE*)*.*

10. A preparation comprising a biological cell prepared *in vitro* by a method comprising the following steps:
1) providing a biological cell comprising a gene on double-stranded DNA;
2) introducing into said biological cell a composition comprising:
- a first CRISPR endonuclease or a nucleic acid molecule encoding the said CRISPR endonuclease;
- a second CRISPR endonuclease or a nucleic acid molecule encoding said second CRISPR endonuclease;
said first and said second CRISPR endonucleases are configured to create single-strand DNA breaks,
- a first single guide RNA (sgRNA), and
- a second sgRNA,
said first sgRNA is configured to hybridize to the sense strand of a genetic element controlling the expression of said gene, and
said second sgRNA is configured to hybridize to the antisense strand of said genetic element controlling the expression of said gene;
3) incubating said cell and said composition, thereby allowing the creation of a single-strand DNA break on the sense strand of a genetic element controlling the expression of said gene and a single-strand DNA break on the antisense strand of the genetic element controlling the expression of said gene, and
4) recovering said cell.

11. The preparation of claim 10, wherein said method is the method of any of claims 7 to 9.

12. A kit for the targeted knockout of a gene on double-stranded DNA in a biological cell, comprising the composition of any of claims 1 to 5 and instructions for delivering the composition to said biological cell so as to knockout the gene.

13. A method of treating a subject afflicted with a disease associated with a mutated gene, comprising administration of a therapeutically effective amount of the composition of any of claims 1 to 6.

14. A nucleic acid molecule, preferably an RNA molecule, comprising a nucleotide sequence which is selected from the group consisting of SEQ ID NOS: 1 to 252.

15. A method of treating a subject afflicted with a disease associated with a mutated gene, preferably a dominant-autosomal disease, alternatively an autosomal-recessive disorder, comprising a step of inhibiting in said subject a genetic element controlling the expression of said gene, preferably knocking-out said genetic element controlling the expression of said gene.
